(19) Europäisches Patentamt / European Patent Office / Office européen des brevets

(11) **EP 3 746 784 B1**

## (12) EUROPEAN PATENT SPECIFICATION

(45) Date of publication and mention
of the grant of the patent:
**01.12.2021 Bulletin 2021/48**

(51) Int Cl.:
*G01N 33/50* [(2006.01)]    *B01L 3/00* [(2006.01)]
*G01N 33/543* [(2006.01)]

(21) Application number: **19701320.4**

(22) Date of filing: **17.01.2019**

(86) International application number:
**PCT/EP2019/051111**

(87) International publication number:
**WO 2019/149531 (08.08.2019 Gazette 2019/32)**

(54) **MICROFLUIDIC DEVICE AND METHOD FOR DETERMINING CELL ELECTRICAL BARRIER PROPERTIES**

MIKROFLUIDISCHE VORRICHTUNG UND VERFAHREN ZUR BESTIMMUNG DER ELEKTRISCHEN BARRIEREEIGENSCHAFTEN VON ZELLEN

DISPOSITIF MICROFLUIDIQUE ET PROCÉDÉ SERVANT À DÉTERMINER DES PROPRIÉTÉS DE BARRIÈRE ÉLECTRIQUE CELLULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.01.2018 GB 201801580**

(43) Date of publication of application:
**09.12.2020 Bulletin 2020/50**

(73) Proprietor: **University Of Southampton
Southampton, Hampshire SO17 1BJ (GB)**

(72) Inventors:
• **MORGAN, Hywel**
  **Southampton SO17 1BJ (GB)**
• **REALE, Riccardo**
  **Southampton SO17 1BJ (GB)**

(74) Representative: **Keilitz Haines & Partner
Patentanwälte PartGmbB
Nigerstraße 4
81675 München (DE)**

(56) References cited:
**WO-A1-2013/086486      WO-A1-2017/096297**

• **TAO SUN ET AL: "On-chip epithelial barrier function assays using electrical impedance spectroscopy", LAB ON A CHIP, vol. 10, no. 12, 8 April 2010 (2010-04-08) , page 1611, XP055536843, ISSN: 1473-0197, DOI: 10.1039/c000699h cited in the application**
• **CORNELIA BLUME ET AL: "Temporal Monitoring of Differentiated Human Airway Epithelial Cells Using Microfluidics", PLOS ONE, vol. 10, no. 10, 5 October 2015 (2015-10-05), page e0139872, XP055569914, DOI: 10.1371/journal.pone.0139872**
• **CORNELIA BLUME ET AL: "Cellular crosstalk between airway epithelial and endothelial cells regulates barrier functions during exposure to double-stranded RNA : Epithelial-endothelial crosstalk in human airways", IMMUNITY, INFLAMMATION AND DISEASE, vol. 5, no. 1, 18 January 2017 (2017-01-18), pages 45-56, XP055569925, ISSN: 2050-4527, DOI: 10.1002/iid3.139**

**Description**

**FIELD OF THE INVENTION**

The invention relates to microfluidic devices and methods for determining cell electrical barrier properties.

**BACKGROUND**

One of the main costs associated with the development of new drugs is the rate of failure of candidates during the clinical trial stage. These costs could be reduced by the adoption of more accurate models with improved reliability at the preclinical stage. Epithelia are among the most biologically relevant features to be modelled, because they are the first tissue that has to be overcome for a drug to penetrate into the inner parts of any organ. Epithelia act as a barrier against the external environment by expressing protein complexes called tight junctions that confer to the tissue selective permeation properties towards ions and macromolecules. Tight junctions are the junctions which form between the abutting membranes of adjacent epithelial cells. The tight junctions form a selectively permeable barrier which restricts the free movement of fluids between the basolateral (inside the body) and apical (environment) sides of the epithelium and are concentrated at the upper apical surface (i.e. polarised expression). The electrical pathways across a confluent cell layer are essentially two-fold: through the cells, which is referred to as the transcellular pathway; and between the cells, which is referred to as the paracelllular pathway which traverses the tight junctions. In a non-confluent cell layer, i.e. one that only partly covers the support membrane, there will additionally be a third pathway through the gaps between individual cells or confluent clusters of cells. Models currently used for the study of epithelia consist of cultures grown on porous supports (with a hole diameter of typically around 0.4 $\mu$m which often leads to them being referred to as nanopores) that facilitate cell polarization, either under static fluid conditions or fluid flow conditions.

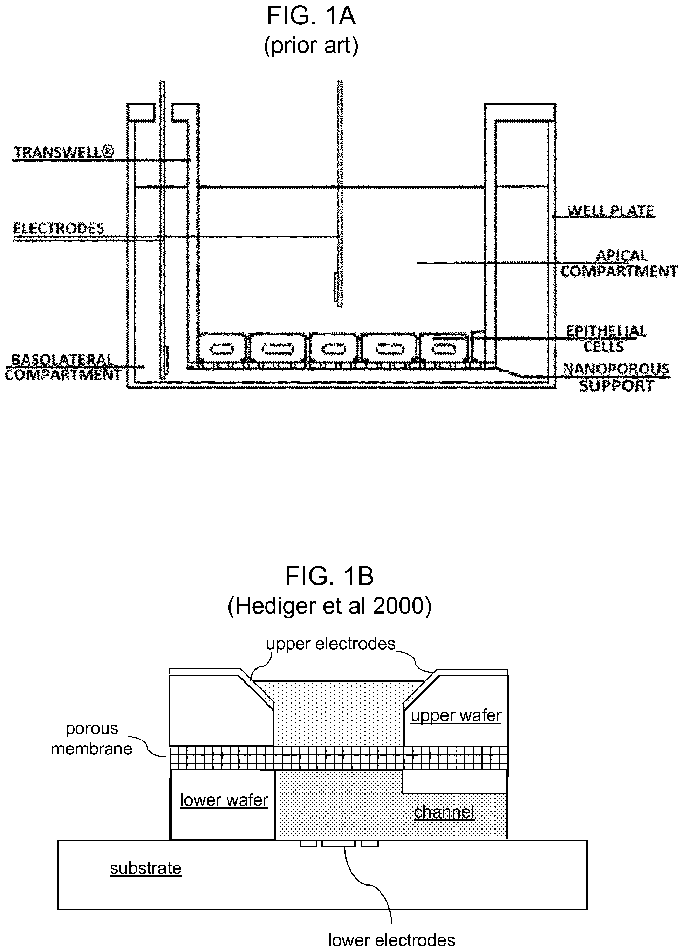

Figure 1A shows a conventional setup for culturing epithelial cells and measuring their trans-epithelial resistance (TER). An example reference for this conventional setup is the control experiments of Odijk et al 2015.

Odijk, van der Meer, Levner, Kim, van der Helm, Segerink, Frimat, Hamilton, Ingber, van den Berg.
"Measuring direct current trans-epithelial electrical resistance in organ-on-a-chip microsystems."
Lab on a Chip vol. 15, issue 3, 7 February 2015, pages 745-52;
DOI: 10.1039/c4lc01219d

A well insert is arranged in a well of a microtiter plate. The well insert has an impermeable cylindrical or frusto-conical wall made of a plastics material which, in the case of a frusto-conical wall, tapers slightly towards the base of the well. The well insert further has a base formed of a porous support membrane which provides a substrate for culturing epithelial cells. (Such well inserts are often called "Transwell" inserts, where "Transwell" is a registered trade mark of Corning, Inc.) The well insert subdivides the well into two compartments, one inside the well insert, referred to as an apical compartment, and one outside the well insert, referred to as a basolateral compartment. First and second Ag/AgCl electrodes are immersed in the apical and basolateral compartments respectively, i.e. the first and second electrodes are arranged either side of the epithelial cells grown on the support membrane. The electrodes are so-called "chopstick electrodes". TER measurements can be performed by the application of a low frequency (e.g. 25 Hz) square wave. TER measurements on a given sample are typically taken in daily or weekly intervals and require the presence of liquid medium in both apical and basolateral compartments.

Figure 1B shows a known setup according to Hediger et al 2000 for culturing epithelial cells using a porous polymeric material support.

Hediger, Fontannaz, Sayah, Hunziker and Gijs
"Biosystem for the culture and characterisation of epithelial cell tissues"
Sensors and Actuators B: Chemical, vol. 63, 20 April 2000, pages 63-73.

Hediger et al describes a device that integrates platinum electrodes above and below a porous polycarbonate membrane which is used as the culture support. The support membrane is sandwiched between crystalline silicon or glass micro-machined wafers arranged on top of a glass substrate. The growth medium for the epithelial cells partially fills the compartment above the support membrane. The Hediger device requires a complex multi-step manufacturing process.

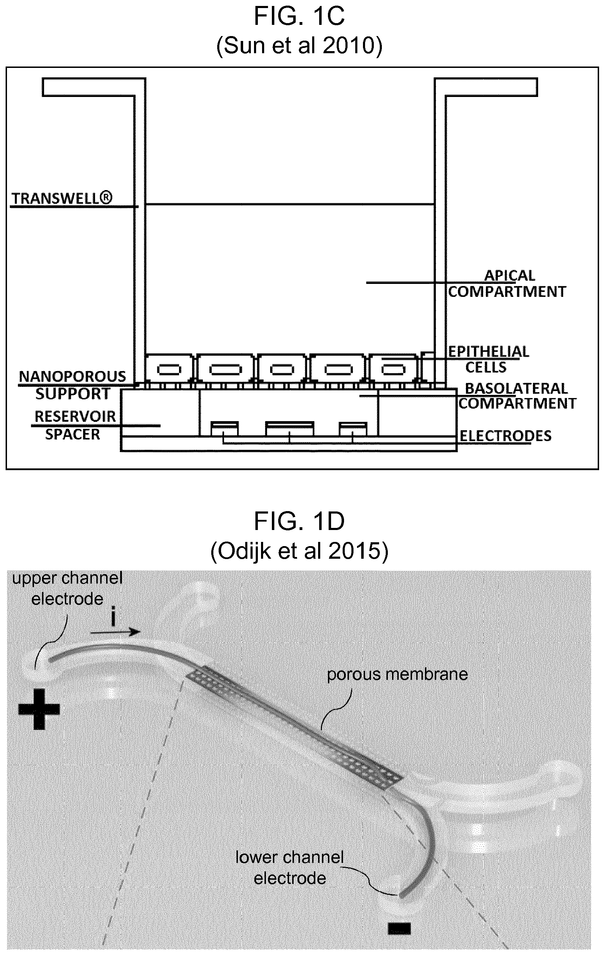

Figure 1C shows a modified setup, according to Sun et al 2010 for culturing epithelial cells

Sun, Swindle, Collins, Holloway, Davies and Morgan
"On-chip epithelial barrier function assays using electrical impedance spectroscopy" Lab on a Chip, volume 10, issue 12, 8 April 2010, pages 1611-1617,
DOI: 10.1039/c000699h.

The setup of Sun *et al* uses a well insert similar to that of Figure 1A. A pair of co-planar, gold-plated copper electrodes are fabricated on a substrate using a printed circuit board approach. An epoxy layer was deposited on the substrate to partially cover the gold-plated electrodes and thereby electrically insulate them, with the epoxy layer having circular apertures arranged to align with the well inserts and thereby form micro-reservoirs that are the basolateral compartments which extend vertically from the substrate to the base of the well inserts. The exposed portions of the electrodes therefore fit within the dimensions of the well insert. The well insert had a diameter of 6.5 mm. The electrical measurement of the epithelial cell culture in this case was an impedance measurement performed over a frequency range of $10^2$ to $10^6$ Hz with an applied voltage of 0.1 V. In an impedance measurement, the electric field penetrates through the pores of the support membrane, where cell-to-cell adhesions (i.e. so-called tight junctions) exist forming the paracellular pathways. At lower frequencies in the $10^2$ to $10^6$ Hz range, due to the existence of the cell membrane, cells behave as insulators. At higher frequencies in the $10^2$ to $10^6$ Hz range, the electric field lines penetrate through the entire cell layer. It is thus possible to assess the status of an epithelial culture by measuring the impedance, because the tight junctions hinder the free movement of conducting ions through the cell layer.

Figure 1D shows the microfluidic set up of Odijk *et al* who compared TER measurements made in a well insert set up similar to Figure 1A with the microfluidic device setup of Figure 1D. In the microfluidic device set up of Odijk *et al* separate microfluidic channels are provided above and below a cell-culturing support membrane. One electrode is arranged in one channel upstream of where the support membrane starts and another electrode is arranged in the other channel downstream of where support the membrane finishes. Odijk *et al* found that the TER measurements in the microfluidic device were artificially high, for example by about 50% in one measurement, compared with comparative well insert measurements. The reason they gave for this distortion in the TER values was that in their microfluidic device most of the current flowed only through the beginning and end parts of the cell construct, whereas in a conventional well insert setup (as shown in our Figure 1A) the conductivity of the culture medium contained in the well insert, i.e. the apical compartment, ensures an almost equal potential drop over the entire cell construct. They explained that in microfluidic channels this is not always the case and current flows only through a part of the cell construct, therefore leading to the higher apparent TER values. Odijk *et al* suggested overcoming this problem by integrating electrodes inside their top and bottom microfluidic channels, so they are directly above and below the support membrane, to provide an equal potential drop over the entire support membrane. However, they stated that there would be major drawbacks of such an approach, namely the electrodes will block the field of view and device fabrication will be more complicated and costly. Instead, they propose as an alternative to correct the distorted TER readings by applying their theoretical model.

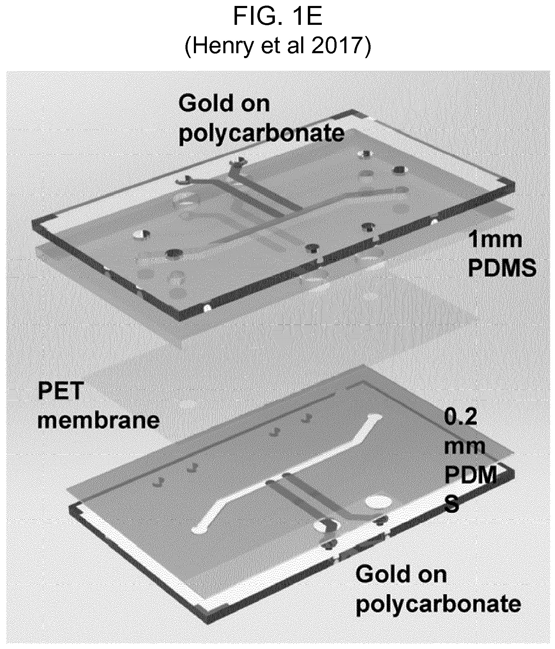

Figure 1E shows the microfluidic set up of Henry *et al* 2017 used to culture epithelial cells at the air liquid interface.

Olivier Y. F. Henry, Remi Villenave, Michael J. Cronce, William D. Leineweber, Maximilian A. Benz and Donald E. Ingber
" Organs-on-chips with integrated electrodes for trans-epithelial electrical resistance (TEER) measurements of human epithelial barrier function."
Lab on a Chip vol. 17, 26 May 2017, pages 2264-71
DOI: 10.1039/c7lc00155j

In the microfluidic device set up of Henry *et al* separate microfluidic channels are provided above and below a cell-culturing support membrane. A pair of electrodes is located on the ceiling of the apical compartment and another pair of electrodes is located on the bottom of the basolateral channel. This system is used to monitor primary epithelial cells. A drawback of this system is the requirement to submerge the air-liquid culture with medium in order to perform the measurement. This means that the TER of cells cannot be continuously measured, neither can the TER be measured during a biological or chemical challenge (e.g. virus, pollen, toxic aerosol, cigarette smoke, ethylene glycol-bis($\beta$-aminoethyl ether)-N,N,N',N'-tetraacetic acid (EGTA), Polyinosinic:polycytidylic acid (Poly(I:C)) ) through the air or basolaterally.

WO 2017/096297 A1 discloses a microfluidic device with a base and a removable fluidic cover through which fluid can perfuse. The base supports a bottom structure and a bottom chamber, above which a membrane is disposed. A top structure is disposed above the membrane and includes a top chamber with an open region.

## SUMMARY OF THE INVENTION

According to a first aspect of the invention a microfluidic device according to claim 1 is provided that has an electrically insulating septum arranged between electrodes in the basolateral compartment to act as a barrier to the electric field between the electrodes that is induced when the electrodes are biased. The septum thus inhibits the direct current path from one electrode to the other through the basolateral medium. More specifically, there is provided a microfluidic device comprising:

a device body;

a porous support extending across an internal volume in the device body to define a partition between an upper, apical compartment that is bounded on its lower side by the upper surface of the porous support and a lower, basolateral compartment that is bounded on its upper side by a lower surface of the porous support and on its lower side by an internal surface of the device body;

an inlet and an outlet arranged in the device body that provide a path for fluid flow of a liquid medium through a flow channel that includes the basolateral compartment;

a first electrode and a second electrode arranged on the internal surface of the device body on the lower side of the flow channel, the first and second electrodes being electrically connected to respective internal ends of respective electrically conductive paths, the electrically conductive paths having respective external ends that provide respective external contacts via which a bias voltage is applicable between the first and second electrodes to create an electric field to induce

an electrical current to flow between the first and second electrodes at least in part via the cell layer, when a liquid medium is present in the flow channel and when a cell layer has been grown on the porous support; and

a septum arranged in the flow channel between the first electrode and the second electrode, the septum extending at least part way between the internal surface of the device body and the porous support, the septum being made of a material that acts as an electrical barrier to the electric field, so that the septum inhibits electrical current flowing directly from the first electrode to the second electrode through the liquid medium and promotes electrical current flowing from the first electrode to the second electrode via the cell layer.

In some embodiments, the flow channel is shaped and arranged to provide a predominant flow direction for the liquid medium passing through it, and the septum is arranged substantially aligned with the predominant flow direction

In some embodiments, the septum extends from the internal surface of the device body to the lower surface of the porous support. This may be defined as the septum terminating sufficiently close to the lower surface of the porous support so that substantially no electrical current flow is possible directly between the first and second electrodes. It does not necessarily mean that the septum is in physical contact with or fixed to the porous support.

According to a second aspect of the invention a microfluidic device according to claim 4 is provided in which the electrodes are comparatively widely spaced apart within the basolateral compartment. More specifically there is provided a microfluidic device comprising:

a device body;

a porous support extending across an internal volume in the device body to define a partition between an upper, apical compartment that is bounded on its lower side by the upper surface of the porous support and a lower, basolateral compartment that is bounded on its upper side by a lower surface of the porous support and on its lower side by an internal surface of the device body;

an inlet and an outlet arranged in the device body that provide a path for fluid flow of a liquid medium through a flow channel that includes the basolateral compartment;

a first electrode and a second electrode arranged on the internal surface of the device body on the lower side of the flow channel, the first and second electrodes being electrically connected to respective internal ends of respective electrically conductive paths, the electrically conductive paths having respective external ends that provide respective external contacts via which a bias voltage is applicable between the first and second electrodes to create an electric field to induce

an electrical current to flow between the first and second electrodes at least in part via the cell layer, when a liquid medium is present in the flow channel and when a cell layer has been grown on the porous support, wherein the fluid flow in the basolateral compartment has a flow direction, and the first and second electrodes have a minimum separation inside the basolateral compartment of 26% of a maximum width of the basolateral compartment. The width of the basolateral compartment may be referenced to the flow direction in the flow channel or the direction of extent of the flow channel in its portion that constitutes the basolateral compartment, i.e. transverse to the flow direction of flow channel direction.

In certain embodiments, the combined area of the first and second electrodes inside the basolateral compartment is at least 20% of the area of the basolateral compartment. This ensures a more uniform current distribution across the cell layer. By comparison, the coplanar electrode design of Sun *et al* had an electrode area equal to 61% of the cell construct width. Further below, we present a first electrode design (Design 1) that has an electrode area equal to 27% of the cell construct width and a second electrode design (Design 2) that has an electrode area equal to 28% of the cell construct width.

The combined area of the first and second electrodes inside the basolateral compartment is, for example, less than 30%, 25%, 20% or 15% of the area of the basolateral compartment. An upper bound to the overall proportion of the compartment covered by electrodes, i.e. the fill factor, expresses the advantageous property of having a large electrode gap.

In certain embodiments, the basolateral compartment has a truncated cylindrical shape.

In certain embodiments, the first and second electrodes have an approximately equal separation across the basolateral compartment. An example of this is represented by our second electrode design (Design 2) described further below which has two parallel electrodes separated by a gap.

It is desirable for the microfluidic device to have a basolateral resistance at least some multiple, e.g. 3, times larger than the apical compartment resistance ($R_b$> 3 $R_a$). For a 100 μm high channel the primary electrodes need to have a gap that is at least 26% of the total width of the cell construct for cultures submerged under 6 mm of medium in the apical compartment or 41% of the total width of the cell construct for cultures submerged under 0.3 mm of medium in the apical compartment. The coplanar electrode design of Sun *et al.* had a minimum gap equal to 7% of the cell construct width. Design 1 has a minimum gap equal to 26% of the cell construct width and Design 2 has a minimum gap equal to 60% of the cell construct width.

It is noted that the porous support and hence the basolateral compartment could be of any shape. A circular porous support, i.e. truncated cylindrical basolateral compartment, is compatible with a conventional Transwell architecture. The porous support could alternatively be rectangular or square to provide a flow channel of constant cross-section in the flow direction. Arbitrary shapes are also technically feasible if unlikely in practice.

The device may further comprise a third electrode and a fourth electrode, as secondary measurement electrodes, arranged in the flow channel, the third and fourth electrodes being electrically connected to respective internal ends of respective electrically conductive paths, the electrically conductive paths having respective external ends that provide respective external contacts via which a bias voltage is applicable between the third and fourth electrodes to create an electric field which, when a liquid medium is present in the flow channel induces an electrical current to flow between the third and fourth electrodes, the third and fourth electrodes being so arranged in the flow channel that current passes between them substantially only via the liquid medium and substantially without passing via a cell layer grown on the porous support.

The role of the secondary measurement electrodes is to make comparative measurements which are used to deduce parameters that are relevant for an equivalent circuit model used for analysing the data obtained from the primary measurement electrodes. The secondary electrodes measure the electrical properties of the medium in the basolateral compartment. Given a knowledge of the geometric cell constant and polarisation impedance (electrical double layer), the conductivity and/or permittivity of the medium may be determined from a measure of the current flowing through the secondary electrodes. Values of the parameters relevant for the electrodes and for the medium determined from the secondary electrode measurements are then used significantly to improve the data fitting procedure for the data obtained from the primary electrodes when using the equivalent electrical circuit. For example, the electrical double layer or constant phase element of the electrode-medium interface can be deduced directly from a measurement using the secondary electrodes. The values can then be used in the equivalent electrical circuit. In a similar manner, the values of the medium conductivity and permittivity are uniquely obtained from the secondary electrodes and used as parameters in the equivalent electrical circuit.

The third and fourth electrodes are preferably arranged in the flow channel outside the basolateral compartment, for example upstream or downstream of the basolateral compartment.

The microfluidic device may further comprise a bubble-trap arranged such that any bubbles that are present in the fluid as the fluid enters the flow channel via the inlet are captured by the bubble trap prior to the fluid flow reaching the basolateral compartment, the bubble trap including a gas-permeable filter.

The gas-permeable filter permits trapped bubbles to dissipate into the atmosphere and also permits the liquid medium to re-equilibrate with the surrounding gaseous environment within the incubator.

In some embodiments, the bubble trap is vertically aligned with the inlet such that any bubbles that are present in the fluid as the fluid enters the flow channel via the inlet are immediately captured by the bubble trap and thus prevented from laterally traversing the flow channel.

In a specific example, the bubble trap is formed by a substantially vertical capillary capped by the gas-permeable filter.

The microfluidic device of any of the above aspects may be incorporated in a system comprising the microfluidic device, for example a device as specified above in combination with a holder for the microfluidic device to facilitate liquid-tight connections for injection and removal of liquids into and out of the microfluidic device and to establish electrical connections to the microfluidic device's electrodes.

Further aspects of the invention relate to methods of using any of the above-specified microfluidic devices: to supply a liquid medium through the flow channel to the basolateral compartment; to grow a cell layer on the porous support; and to use the electrodes to take measurements of the cell layer at different stages of its growth.

The electrode designs we have developed for measurement of the electrical properties of epithelial cell cultures make it possible to make electrical measurements with relatively small amounts of electrolyte in the apical compartment. As described below, to determine the optimum electrode design an equivalent electric circuit was developed. This was used to study the effects of each circuit element on the sensitivity. An optimal electrode geometry maximizes the basolateral resistance and minimizes the apical compartment resistance. The best-performing electrode designs we developed had a parallel gap between two equal-area electrodes arranged either side of the circular area covered by the support membrane at the base of the well insert or cylindrical microfluidic device chamber. Finite element analysis was used to optimize the geometrical parameters to gain the best compromise between sensitivity and uniform current distribution of the current flowing through the culture. The design has a greater sensitivity and a more uniform current density distribution across the support membrane compared to the prior art concentric electrode design of Sun *et al.* We found that performance is further improved by arranging an electrically insulating septum between the electrodes.

The effect of the volume of electrolyte in the apical compartment on the measurement was also analysed. The sensitivity increases logarithmically with increasing electrolyte volume. Impedance was used to monitor the development of an epithelial barrier (cell culture resistance $R_c$, culture capacitance $C_c$ and apical resistance $R_a$) with a small volume of liquid in the apical compartment (10 $\mu$l, leading to an electrolyte height of 0.3 mm).

The proposed microfluidic devices may be used, for example, to study primary human airway cells cultured at the air-liquid interface. The minimum height of electrolyte required for the measurement is within the range of the amount of mucus physiologically produced by differentiated primary human airways cells.

A modular design for the microfluidic device is described that is relatively simple to manufacture.

The microfluidic device is suitable for integration in organ-on-chip technologies, where it could be used to perform real-time monitoring of electrical tissue properties, either at the air-liquid interface or under submerged conditions depending on whether the apical compartment is filled with air or an aqueous liquid.

## BRIEF DESCRIPTION OF THE DRAWINGS

This invention will now be further described, by way of example only, with reference to the accompanying drawings.

Figure 1A shows a conventional setup for culturing epithelial cells using a porous polymeric material supports.

Figure 1B shows a known setup according to Hediger et al 2000 for culturing epithelial cells using a porous polymeric material support.

Figure 1C shows a known setup according to Sun et al 2010 for culturing epithelial cells using a porous polymeric material support.

Figure 1D shows a known microfluidic set up according to Odijk et al 2015 for culturing epithelial cells using a porous polymeric material support.

Figure 1E shows a known microfluidic set up according to Henry et al 2017 for culturing epithelial cells using a porous polymeric material support.

Figure 2A is a perspective drawing of a proposed measurement platform according to an embodiment of the invention.

Figure 2B is a detailed perspective, vertical section drawing showing parts of the measurement platform of Figure 2A.

Figure 3A is a schematic perspective drawing of the microfluidic chip according to an embodiment of the invention designed for real-time monitoring of epithelial cell growth and cell function.

Figure 3B is a schematic drawing of disassembled principal components of the microfluidic chip of Figure 3A in which electrodes and an integrated bubble trap are visible.

Figure 3C is a schematic vertical section through Figure 3A

Figure 3D is a vertical section through the section plane of Figure 3B showing the electric field distribution in the system during an impedance measurement.

Figure 3E is a schematic drawing of the principal components of a system including the microfluidic chip of Figure 3A.

Figure 4 is a schematic vertical section of the same section as Figure 3C showing an equivalent electrical circuit (EEC).

Figures 5A and 5B are graphs of electrical impedance amplitude/modulus and phase as a function of frequency during growth of a submerged, epithelial cell culture.

Figure 6 is a graph showing results of a frequency-dependent model for the impedance magnitude.

Figure 7 is a graph showing sensitivity, S, as a function of basolateral resistance, Rb, for cultures with three different apical compartment resistances, Ra, of 500 k$\Omega$, 1000 k$\Omega$ and 1500 k$\Omega$.

Figure 8 shows various example of primary electrodes geometries (Designs 1 and 2) as well as the prior art geometry used by Sun *et al* 2010 (Design 0).

Figure 9 is a bar chart comparing sensitivity and basolateral resistance for each of the electrode designs shown in Figure 8.

Figure 10 is a graph of sensitivity against electrode gap size for the electrode geometry of Design 2.

Figure 11 shows example geometries corresponding to Designs 1 to 2, but modified by inclusion of an insulating septum shown by the lines.

Figure 12 shows results of finite element simulations for different variants of Design 2 and Design 2s with an insulating spacer providing a 1 mm gap (top), 3 mm gap (middle) and 3 mm gap with septum (bottom).

Figures 13A and 13B are graphs of electrical impedance modulus and phase as a function of frequency for a confluent epithelial culture with different amounts of electrolyte in the apical compartment as specified by height and also in brackets volume.

Figures 14A and 14B is a graph showing results of a measurement of sensitivity as given by impedance modulus and phase angle as a function of the amount of electrolyte in the apical compartment as specified by height and also in brackets volume.

Figure 15A is a graph of resistance versus time showing the results of monitoring growth of epithelial cultures with different heights of electrolyte in the apical compartment.

Figure 15B is a graph of capacitance versus time for the same results as Figure 15A.

Figure 16 is a bar chart of fitted apical compartment resistance values, Ra, for different volumes of medium in the apical compartment.

Figure 17 is a bar chart of fitted resistance values, Rc, of a primary culture of epithelial cells before and after disruption with EGTA, measured with either the standard system, submerged, or at the air-liquid interface.

## DETAILED DESCRIPTION

In airways of vertebrates, such as mammals, birds or reptiles, the epithelium is exposed to air on one side and liquid on the other. This Air-Liquid Interface (ALI) is a physiological condition that must be recreated *in vitro* in order to culture airway tissue, since the ALI is required to polarize and differentiate the cells which is pre-condition of culturing. The electrical resistance of the epithelial barrier formed at the ALI interface cannot be measured *in situ* using the classical trans-epithelium electrode configuration of Figure 1A, because of the absence of a conducting electrolyte above the cell/tissue construct. This problem can be overcome by temporarily filling the air compartment with a liquid medium, but this is a disruptive and labour intense process.

Herein is described a simple system for *in-situ* electrical impedance spectroscopy (EIS) of epithelial cells cultured on porous supports which can be applied to fully submerged systems or for cells grown at the air-liquid Interface. The system can be used to measure the electrical properties of tissues cultured on commercial static supports (Transwell®) or can be integrated into microfluidic devices for real-time monitoring of epithelia.

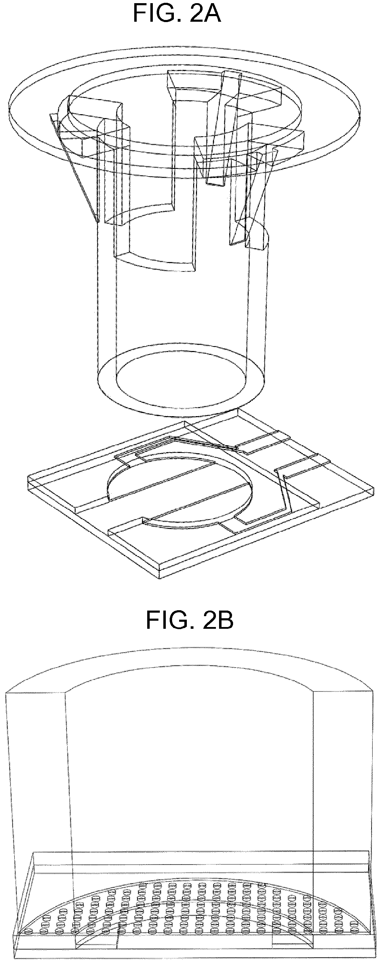

Figure 2A is a perspective drawing of a proposed measurement platform according to embodiments of the invention including a measurement chamber. First and second, thin film electrodes are fabricated on a planar substrate. The electrodes are made of platinum and the substrate is made of a glass, such as a silica or silicate glass. Example silicate glasses include borosilicate, germanosilicate and phosphosilicate. A porous polymeric material support bearing tissue construct is placed on top of the primary electrodes for measurement.

Figure 2B is a perspective drawing of the measurement chamber which is part of the platform shown in Figure 2A. A spacer extending around and on top of the primary electrodes defines the volume in the basolateral compartment.

First and second platinum electrodes are provided which lie on the same plane, i.e. are co-planar, as shown in Figure 2A and Figure 2B. Instead of platinum, other materials could be used for the electrodes, for example the electrodes could be made of platinum black (instead of platinum metal) or gold or a conducting polymer or a silver/silver chloride combination. When a potential is applied between the primary electrodes, current flows both below and through the cell layer of epithelial cells grown on the well insert's porous support membrane. The amount of current flowing through the cell layer depends on the electrical properties of the cell layer. For measurement of TER using a well insert, the tissue culture is positioned over the primary electrodes. A typical duration for a single TER measurement is 30 seconds.

These electrodes enable continuous measurements of TER over many weeks (the Pt is nontoxic unlike Ag/Ag-Cl). A thin polymer spacer defines the volume in the basolateral compartment.

The medium in the apical compartment is static, but the cell culture medium in the basolateral compartment underneath the support membrane can flow, since the basolateral compartment is a section of a micro-fluidic channel through which the cell culture medium passes.

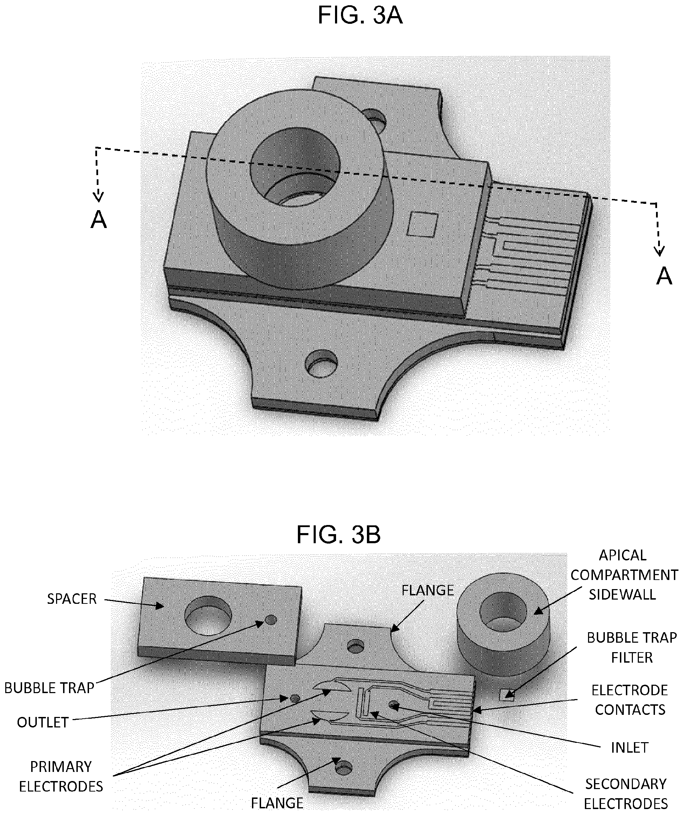

Figure 3A and Figure 3B are schematic perspective drawings of a microfluidic chip designed for real-time monitoring of epithelial cell growth and cell function.

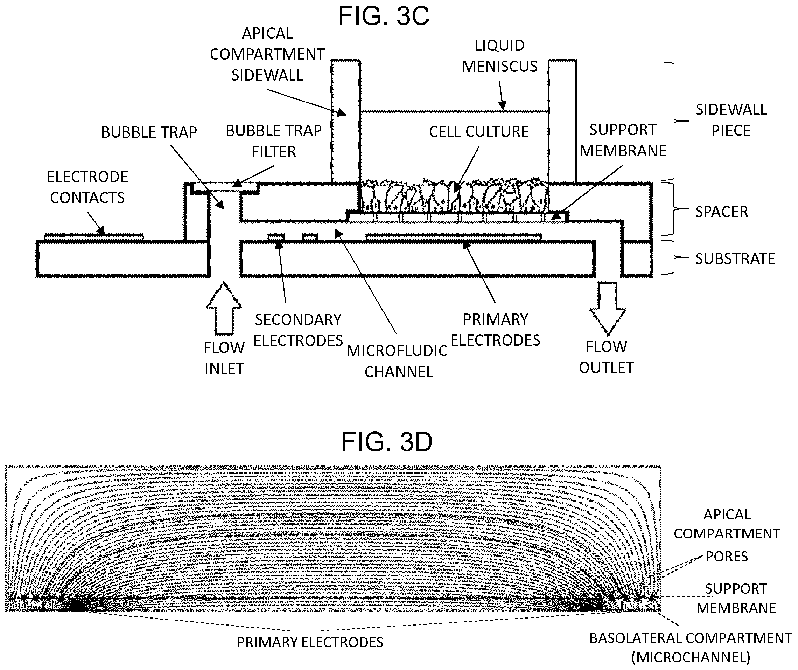

Figure 3C is a schematic cross-section through section A-A of Figure 3A showing the path of the microfluidic channel defined above the electrodes. Inlet and outlet ports are present in the glass chip. An additional pair of electrodes is added to measure the electrical conductivity of the fluid in the channel and monitor variation of its temperature.

Referring to Figure 3A and Figure 3B and also Figure 3C, it can be seen that the microfluidic chip has various structural components. The first component is a planar substrate having an upper surface on which the primary and secondary electrodes and their contacts are fabricated. The substrate also has first and second through holes constituting an inlet (port) and an outlet (port) via which fluid is flown into and out of the chip. The substrate has side flanges, each illustrated with a single through hole, to aid attachment of the microfluidic device to a suitable holder during assembly and use. The second component is a planar spacer which has a bubble trap formed of a through hole vertically aligned with the inlet on the substrate and a large circular through hole arranged vertically above the primary electrodes to define the basolateral compartment. On the lower surface of the planar spacer there is an indented surface (see Figure 3C, but not visible in Figure 3A and Figure 3B) which forms inlet and outlet portions of the fluidic channel. The third component is a cylindrical body that forms the sidewalls of the apical compartment, the inner diameter of the cylindrical body is matched to the inner diameter of the large through hole in the spacer. A fourth component shown in Figure 3B is a gas-permeable filter for the bubble trap which is arranged as shown in Figure 3C on the upper surface of the spacer at the upper end of the bubble trap through hole. A fifth component shown in Figure 3C is the support membrane that is arranged on the indented, lower surface of the spacer to form the divider between the apical compartment above and the basolateral compartment lying in the microfluidic channel below. The porous support provides the matrix on which the cell culture may grow as schematically illustrated in Figure 3C. The bubble-trap through hole thus provides a capillary for the bubbles. It is noted that the capillary need not be a simple through hole between the upper and lower surfaces, although that is an easy construction, but could follow some other, non-straight path. For example one or more bends could be provided, e.g. so that the capillary emerges from a side surface of the spacer rather than at the top. This may for example be desirable from an operator access perspective in some cases.

It will thus be appreciated that a modular construction has been adopted in which the different components are easy to manufacture and assemble. Moreover, variations in the design are easily achievable, e.g. modification of the electrodes involves only changing the patterning on the upper surface of the substrate.

Regarding the electrodes, these comprise two pairs a pair of primary electrodes and a pair of secondary electrodes. The primary electrodes are arranged on the upper surface of the substrate in the microfluidic channel directly below the apical compartment and support membrane, so that when a liquid medium is present in the flow channel and when a cell layer has been grown on the porous support, a voltage applied between the primary electrodes induces an electrical field and hence current to flow between them via the cell layer. That is at least some component of the induced current flows through the cell layer, although some other component of the induced current may flow directly through the liquid medium from one primary electrode to the other, so be insensitive to the cell layer. Each of the primary electrodes feeds out to respective external contacts (visible in each of Figure 3A, Figure 3B and Figure 3C) to allow them to be connected to an external circuit for biasing and measurement. In addition to the primary electrodes, secondary electrodes are provided in the flow channel, but outside the basolateral compartment, so that current induced by biasing the second electrodes passes between them substantially only via the liquid medium and substantially without passing via a cell layer grown on the porous support. The measurements obtained from the secondary electrodes are thus completely insensitive to the presence of the cell layer, and hence provide a control measurement compared to the measurements from the primary electrodes. The secondary electrodes are illustrated as being arranged upstream of the basolateral compartment as referenced to the flow direction. In other words, an upstream arrangement means positioning between the flow inlet and the support membrane. In an alternative embodiment (not illustrated) the secondary electrodes are arranged downstream of the basolateral compartment. In other words, a downstream arrangement means positioning between the support membrane and the flow outlet. Figure 3D is a plot showing the electric field in the system during the primary electrode measurement. A part of the current flows through the tissue construct and a part flows below it.

Figure 3A and Figure 3B show a microfluidic device made from a polymer material or a glass or another suitable chemically inert material such as a crystal (e.g. Si). A suitable polymer material is polymethyl methacrylate (PMMA) which is included in the following list of suitable polymer materials:

1. Polymethyl Methacrylate (PMMA), e.g. trade name Röhm VQ105, Perspex
2. Polystyrene (PS)
3. Polypropylene (PP)
4. Polyethylene (PE)
5. Cycloolefin (co-)polymer (COP), e.g. trade names Zeon Zeonex, Ticona Topas, Mitsui APEL, Japan Synthetic Rubber Arton
6. Styrene-acrylonitrile copolymer (SAN), e.g. trade name BASF Luran
7. Polyamide (nylon)
8. Polyimide (PI)
9. Polycarbonate (PC)

Any suitable glass or any of the above-listed polymer materials may be used for whole or part of the structure of the microfluidic devices, either in surface treated or untreated (natural) form, to provide the desired combination of biological, chemical, optical and/or fluidic properties.

The microfluidic device has a porous support membrane on which the cells are grown which is similar to the base of a conventional well insert. The microfluidic device has a microfluidic channel which is in fluid communication with an inlet port, an outlet port, and is bounded at least in part on one side by the support membrane which separates the microfluidic-channel forming basolateral compartment from the apical compartment. The electrodes are initially fabricated on a substrate. A spacer is arranged on the substrate to define the microfluidic channel in which the electrodes are at least partially situated and through which the cell growth medium can flow.

As shown in Figure 3C and previously mentioned, a bubble trap is integrated in the device, vertically aligned with the inlet port. The bubble trap consists of a vertical bore capped with a gas-permeable filter. When a gas bubble is present in the inlet stream, it is captured from the medium by the trap due to its higher buoyancy. The bubble then travels through the trap and is removed through the gas-permeable filter. This bubble trap can operate in continuous flow and does not require maintenance. Examples of materials that could be used as gas-permeable filters are: polydimethylsiloxane (PDMS) and polytetrafluoroethylene (PTFE).

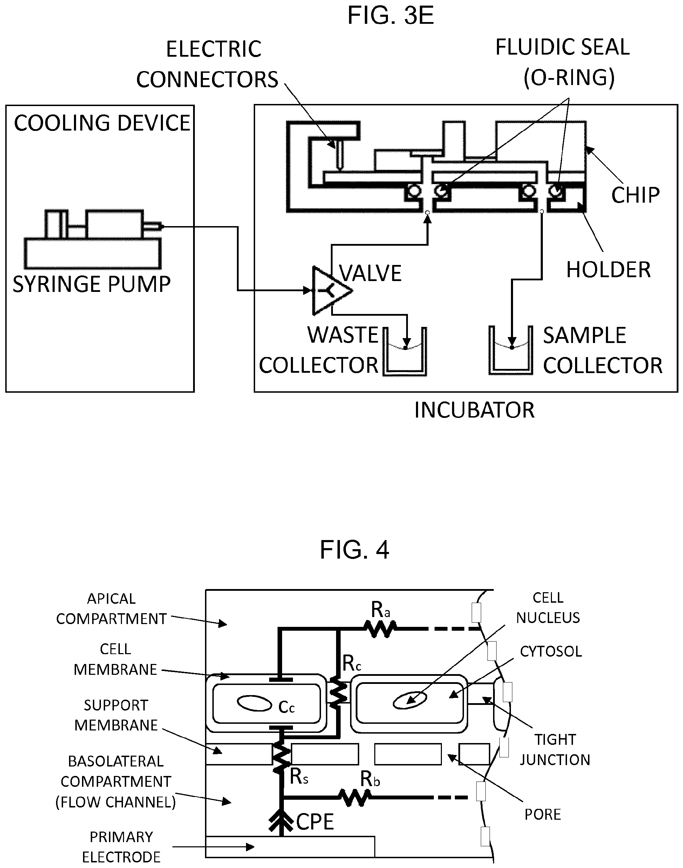

Figure 3E is a schematic drawing of the experimental setup of a system incorporating the above-described microfluidic chip. The main system sub-assemblies are an incubator which accommodates the microfluidic chip and a cooling device which accommodates a pump, such as a syringe pump, for supplying the liquid cell growth medium to the microfluidic chip. The incubator is shaped and dimensioned to releasably accommodate the microfluidic device. The syringe pump has a reservoir for containing the cell growth medium. The syringe pump is kept refrigerated, e.g. at 4°C, by the cooling device. The cooling prevent degradation of growth medium (for example components in the growth medium such as proteins or retinoic acid) which would occur if the growth medium were held at room temperature. The incubator is operable to maintain the microfluidic chip and other components accommodated therein to a suitable temperature, such as 37°C. The microfluidic chip is clipped into the holder which provides both electric connections (for example spring-loaded connectors, such as Pogo pins, or Samtec-SEI connectors) and fluidic connections (for example access ports with o-rings) between the microfluidic chip and wider system components. Seals in the form of o-rings form liquid-tight seals between the chip inlet and outlet and the holder. A liquid-carrying capillary connects the syringe pump with the chip inlet via a valve. The holder is shaped and dimensioned to releasably hold the microfluidic device so that electrical and fluidic connections are formed to external electrical and fluidic components. The valve is switchable to divert the liquid flow from the syringe pump either to the chip inlet or a liquid waste collector. The chip outlet is connected via another capillary to a liquid sample collector. The gases dissolved in the growth medium (i.e. oxygen and carbon dioxide) reach equilibrium conditions in the bubble trap upstream the cell culture chamber, where they can dissipate into the atmosphere through the gas-permeable filter.

As shown in Figure 3C and described above, an additional, secondary pair of electrodes is provided adjacent to the support membrane to measure certain electrical parameters that can be used for analysing the impedance measurement made with the primary electrode pair according to the equivalent circuit described below. The secondary electrode pair is located preferably upstream from the cell culture chamber at a distance such that the generated electric field does not interact with the cell layer. The secondary electrode pair is used to independently measure the electrical properties of the cell growth medium in the basolateral compartment. This secondary measurement is performed either simultaneously or a short time before or after the primary measurement and is used to compensate the primary measurement for temperature fluctuations or electrode fouling. In one example the secondary measurement is used to determine variations in the medium conductivity by assuming a purely resistive behaviour. For example, a measurement gives the conductivity as follows: $Z_2 = K_2 * \rho_{GM}$, where K is the geometric cell constant, $\rho$ the conductivity, the subscript 2 refers to the secondary electrode parameters and the subscript GM refers to the growth medium) which are then used to compute the basolateral resistance of the primary measurement $R_b = K_b * \rho_{GM}$. The amount of electrode fouling over time can be assessed from a full frequency spectrum of the secondary electrodes to determine the CPE.

The electrical properties of this system were analysed using an equivalent electric circuit (EEC) and finite element analysis (FEA). The simulation results were used to optimise the sensitivity of the electrode configuration, thereby enabling real time monitoring of the epithelial barriers formed by a human bronchial epithelial cell line (16HBE14o-) and monitoring of the disruption of an epithelial barrier formed by primary cells. Examples of other cells that could be used in this device are:

1. Madin-Darby canine kidney (MDCK, MDCK-II)
2. Human Caucasian colon adenocarcinoma (Caco-2)
3. Human colorectal adenocarcinoma (HT-29)
4. Human bronchial epithelium (BEAS-2B)
5. Human Umbilical Vein Endothelial Cells (HUVEC)
6. Epithelium-endothelium and other co-cultures

## EQUIVALENT ELECTRIC CIRCUIT MODEL

Figure 4 is a schematic cross-section through section A-A of a part of what is shown in Figure 3A. The schematic illustration in Figure 4 is an overlay of the equivalent electrical circuit (EEC) onto the tissue construct of the cell layer. The EEC comprises polarizable electrodes (Constant Phase Element, CPE), the basolateral medium resistance in the channel (Rb), the polymeric porous cell support (Rs), the cell layer (Rc, Cc) and the medium in the apical compartment (Ra). The EEC corresponds to the two parallel current pathways: one along the bottom fluid channel and another through the cell layer. Figure 4 also schematically depicts the (nano)pores in the porous support.

The EEC is based on the lumped parameter circuit reported by Sun *et al.* The model includes the electrodes, the basolateral medium in the channel, the polymer cell support, the cell culture and the medium in the apical compartment. As shown in the figure, there are two parallel pathways for the electrical current, one along the basal channel and a second through the porous support, the cell culture and the apical compartment. The electrodes are polarisable and were modelled as a constant phase element (CPE). The cell layer was modelled as a parallel resistor-capacitor combination. The resistor represents the current flowing between the cells and through the tight junctions (Rc) and the capacitor describes the charging current flowing through the cell membrane and the cytoplasm (Cc). The reactive components of the polymer cell-support and of the medium in both the apical compartment and the basolateral channel are negligible in the experimental frequency range ($10^2$ Hz to $10^5$ Hz) and are neglected. The resistive components of the polymer support (Rs) and the medium in the apical compartment (Ra) are lumped together as a single resistor (Rs+Ra). The total impedance is not linearly dependent on the tissue construct parameters due to the presence of the other elements in the system. The equivalent electric circuit is used to fit experimental impedance data and extract quantitative information for all the parameters. Typical values of the fitted parameters are reported in Table 1.

Table 1 Typical values of fitted parameters

| PARAMETER | PHYSICAL MEANING | TYPICAL VALUE |
|---|---|---|
| CPE Coefficient | Electrode Impedance | 5000 [Ohm] |
| CPE Exponent | Electrode polarizability (resistive or capacitive) | 0.51 |
| $R_b$ | Basolateral compartment resistance | 8000 [Ohm] |
| $R_a$ | Apical compartment resistance | 500 [Ohm] |
| $R_c$ | Construct resistance | 0-1500 [Ohm] |
| $C_c$ | Construct capacitance | $10^{-8}$-$10^{-7}$ [F] |

**SUBMERGED EPITHELIAL CELL CULTURES** - **experimental**

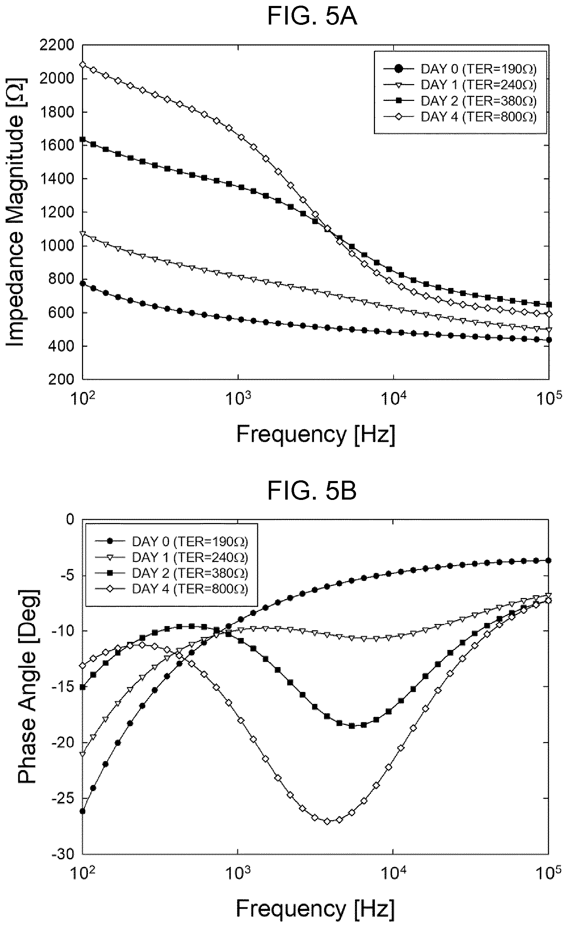

Figures 5A and 5B are graphs of electrical impedance amplitude/modulus and phase as a function of frequency during growth of a submerged, epithelial cell culture. The baseline (day 0) is dominated by low frequency electrode polarisation. As the cells grow in the barrier region (days 1-4), the impedance amplitude increases at lower frequencies and a relaxation is observed in phase angle at higher frequencies. At higher frequencies, the current flows predominantly directly through the cells and the impedance is independent of the status of tight junctions.

EIS measurements were performed daily on epithelial cells growing on commercial Transwell supports. An example of the frequency-dependent behaviour of such a cell culture is shown in Figures 5A and 5B. The baseline impedance spectrum at day zero shows that the reactive component of the impedance dominates at low-frequencies due to electrode polarisation.

This leads to an exponential increase in the impedance magnitude at low frequency. As the cell barrier forms, the TER increases and the cell membrane capacitance, Cc, must be introduced into the model. At frequencies greater than 10 kHz the cell membrane capacitance, Cc, is short circuited, therefore the current flows through the cell cytoplasm and the impedance magnitude will be almost independent of the cellular barrier. At frequencies lower than 1 kHz the cell membrane capacitance, Cc, acts as an open circuit, therefore the current flows in between the cells and the impedance magnitude increases as intercellular tight junctions are formed. The frequency-dependent coupling between the signal and the culture leads to the formation of changing phase angle as shown in Figure 5B.

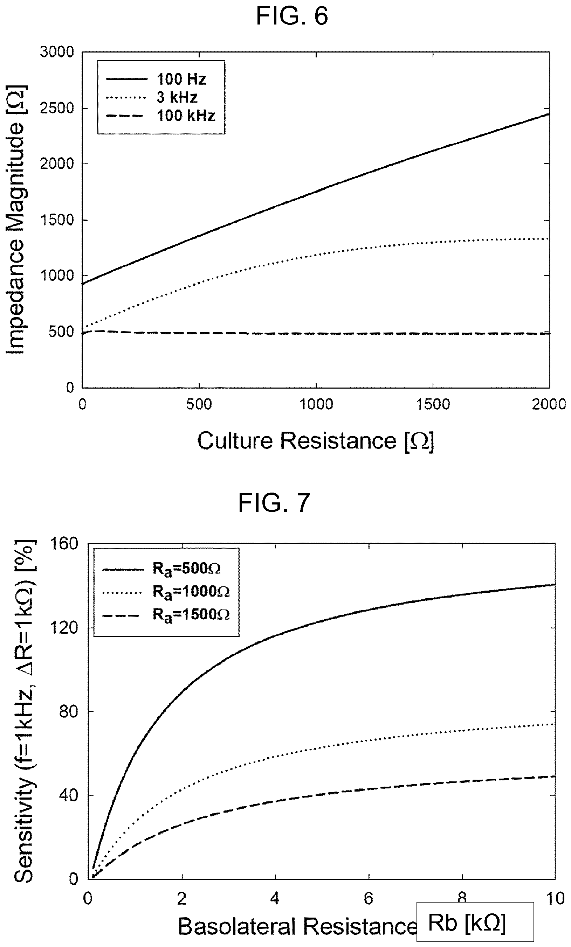

Figure 6 is a graph showing results of a frequency-dependent model for the impedance magnitude. At 100 Hz the impedance is directly proportional to the culture medium resistance, whereas at 100 kHz it is constant. In the 3-7 kHz range the measurement has a transition from one behaviour to the other. Figure 6 provides a further analysis of the frequency-dependent behaviour of the system. At low frequencies (100 Hz) the impedance magnitude is directly proportional to the culture medium resistance, whereas at high frequencies (100 kHz) it is not affected by the culture medium. At intermediate frequencies (3-7 kHz) there is a transition from one behaviour to the other.

Figure 7 is a graph showing sensitivity, S, as a function of basolateral resistance, Rb, for cultures with three different apical compartment resistances, Ra, of 500 kΩ, 1000 kΩ and 1500 kΩ. The graph shows that sensitivity is affected by both the basolateral and apical compartment resistances. The values for the impedance are constrained in the range [(Ra//Rb ), Rb]. For small Rb and big Ra, small changes in the culture resistance can saturate all the measurement range. The sensitivity is maximized for Ra << Rc << Rb, where Rc is the cell layer's resistance.

**ELECTRODE OPTIMIZATION**

The parameters that characterize the cell layer are the tight junction resistance Rc and the cell layer capacitance, Cc. The total impedance is not linearly dependent on these parameters due to the presence of the parallel basolateral resistor Rb and the series resistor Ra. To analyse the effects of these parameters on the measured impedance, a sensitivity can be defined as:

$$S_{R_b} = \frac{|Z_{1kHz}(R_c = 1000\Omega)| - |Z_{1kHz}(R_c = 0\Omega)|}{|Z_{1kHz}(R_c = 1000\Omega)|}$$

The sensitivity of the measurement to variations in the cell layer depends on the values of the basolateral compartment resistance, Rb, and on the apical compartment resistance, Ra, as shown in Figure 7. The highest sensitivity is obtained when the basolateral resistance, Rb is maximum so that the current in the apical compartment is maximum, i.e. Ra is low. If the basolateral resistance is small most of the current will flow in the channel and the impedance measurement will be independent of the resistance of the cells. If the basolateral resistance is much bigger than the culture resistance it will effectively behave as an open circuit and the optimal condition is obtained. The values for the impedance are constrained in the range [(Ra//Rb), Rb] (where // indicates the parallel connection between the two resistances): the lower boundary is in the absence of cells (Rc=0); the upper boundary is the ideal case with Rc→∞.

The electrode geometry needs to maximise the basolateral resistance with minimum electrode polarization effects. Electrode polarization can be reduced by increasing the size of the electrode or by increasing the specific area with surface treatments, for example by platinum black coating. The geometrical parameters that affect the basolateral resistance are the reservoir height and the distance between the electrodes.

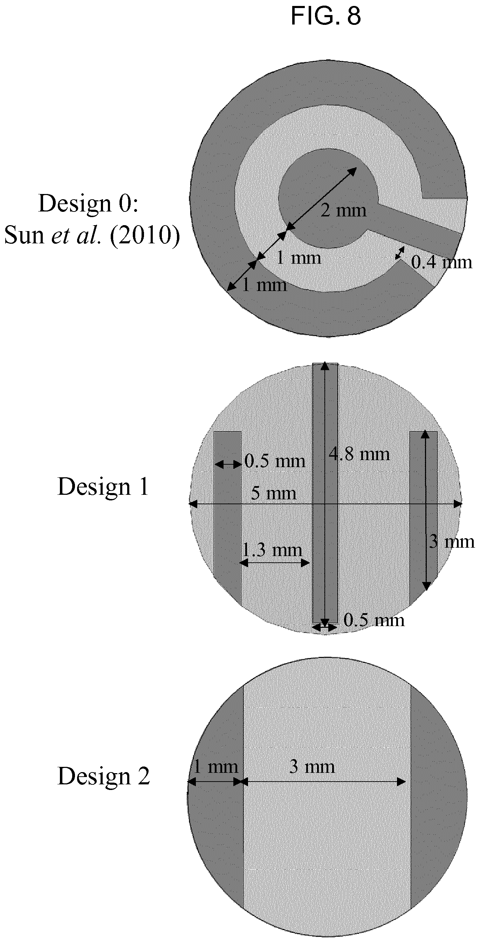

Figure 8 compares the design of Sun *et al* with four new designs. The design geometric parameters are as follows:

Design 0 (Sun *et al*): Concentric electrodes: dot radius = 1 mm, ring inner radius = 2 mm, ring outer radius = 3 mm.

Design 1: Interdigitated straight electrodes: width = 0.5 mm, gap = 1.3 mm.

Design 2: Two parallel lines: width = 1 mm, curvature radius = 2.5 mm and gap = 3 mm.

The design of Sun *et al* has two concentric electrodes. Design 1 has three interdigitated straight electrodes. Design 2 has two circular parallel strips.

Finite element analysis (FEA) was used to numerically evaluate the electrical characteristics of these different electrodes design. The percentage of the total current flowing through the culture medium was used as an indicator of sensitivity. When the ratio is 100% all the current flows through the cell layer and the sensitivity is maximum; if the ratio is 0% no current goes through the cells. The numerical integrals for the current density are evaluated at the electrode plane (z=0) and at the cell culture plane (support membrane) (z = 0.1 mm). Another way to assess the sensitivity is to measure the basolateral resistance. Simulated values for different geometries are calculated by dividing the applied potential by the numerical integral of the total current.

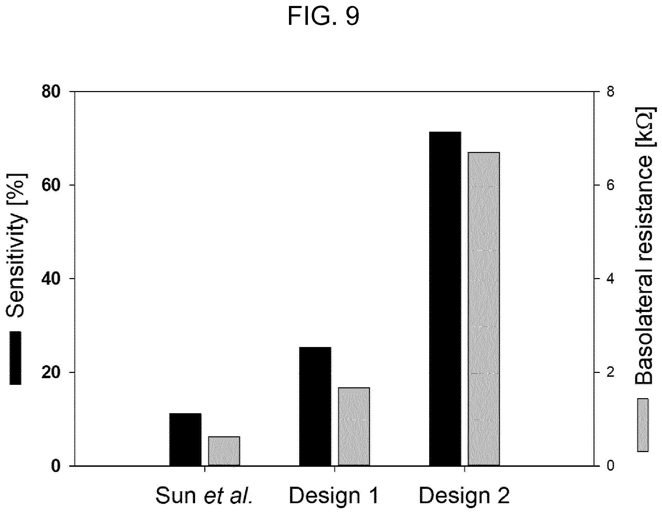

Figure 9 is a bar chart comparing sensitivity and basolateral resistance for the different electrode designs of Figure 8 according to a finite-element-analysis (FEA) electric simulation. There is a correlation between sensitivity and the basolateral cell resistance. The simulations results are shown in Figure 9: there is a large variation in the percentage of current across the design. There is a correlation between the current flowing through the cells and the basolateral cell resistance: the best designs have the largest distance between the electrodes. The optimal design is Design 2, which has a basolateral resistance of 6.7 kΩ and sensitivity of 71.3%. In comparison, the original concentric electrode design has a basolateral resistance of 620 Ω and a sensitivity of 11.1%. The new designs are significantly more sensitive than the Sun *et al* design.

Another parameter that should be considered is the uniformity of the current across the tissue construct.

The current density standard deviation in the plane was calculated to determine the uniformity. The resulting standard deviations are 6.28 A/m2 (58% of the mean) for the concentric electrodes design and 4.63 A/m2 (9% of the mean) for Design 2 indicating a greater uniformity of the current density across the porous support membrane in Design 2.

The performance of the new electrode designs depends on the gap size. To determine the optimum gap, the ratio between current flowing through the cell layer and the total current in the device was determined. Current density uniformity was also analysed.

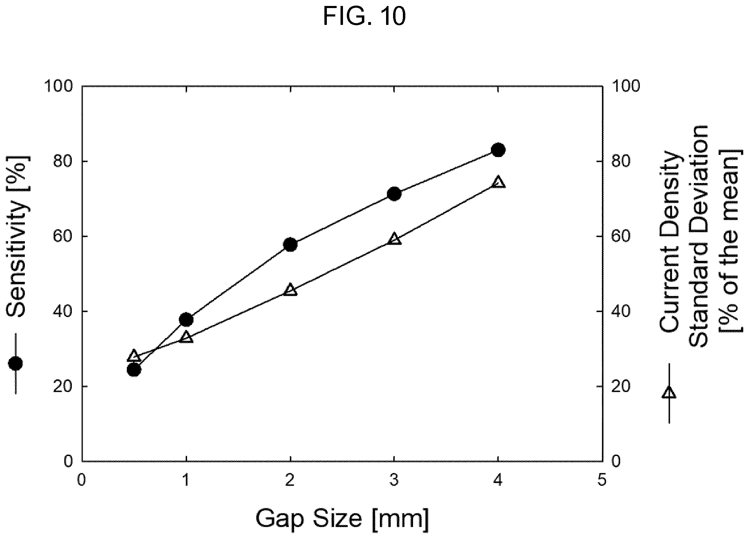

Figure 10 is a graph of sensitivity against electrode gap size for Design 2. As the electrode gap is increased, the basolateral resistance is increased, so that more of the current flows through the cells and sensitivity is improved. However, increasing the gap also results in a decrease in the electrode area which leads to a less uniform current distribution. The optimum gap for Design 2 is around 3 mm (e.g. 3±0.5 mm), with a 3 mm gap resulting in 71.3% of the total current flowing through the cell layers, with a current density standard variation of 4.63 A/m2 (59% of the mean).

It is possible to further increase the basolateral resistance of the system and therefore its sensitivity by adding an electrically insulating barrier between the primary electrodes.

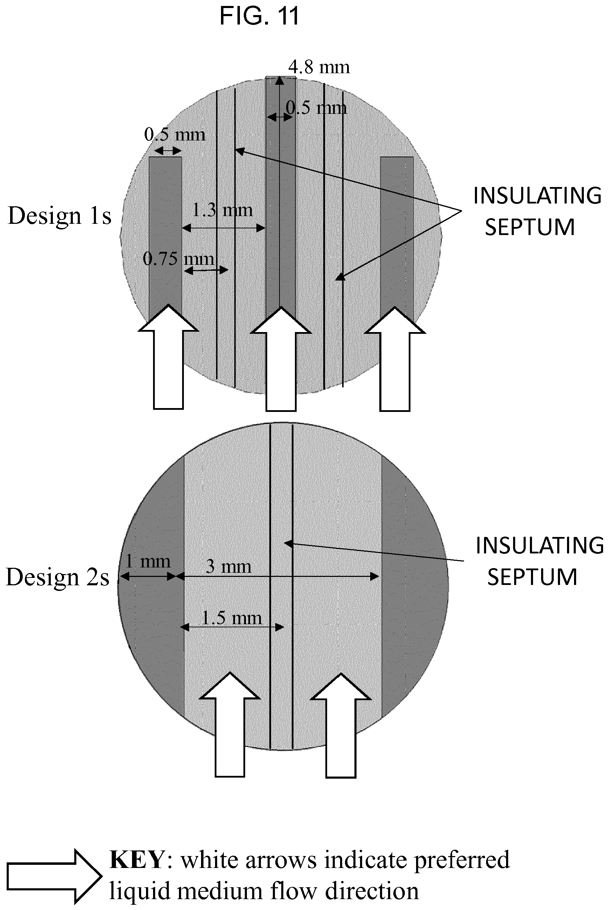

Figure 11 shows such a design approach as a modification of Design 2, which we refer to as Design 2s. However, it is noted that a similar approach could also be taken to modify Designs 1, or indeed the design of Sun et al. An insulating obstacle provides a barrier to current flowing through the basolateral compartment and forces more of the electrical current to pass through the cell layer. An upstanding insulating barrier is provided between the primary electrodes extending vertically between the substrate and the underside of the support membrane, or close thereto. We refer to the insulating barrier as a septum. If the height of the obstacle is equal to height of the reservoir spacer, i.e. the full height of the basolateral compartment, substantially no current can flow across the channel, Rb→∞ and the sensitivity is maximised.

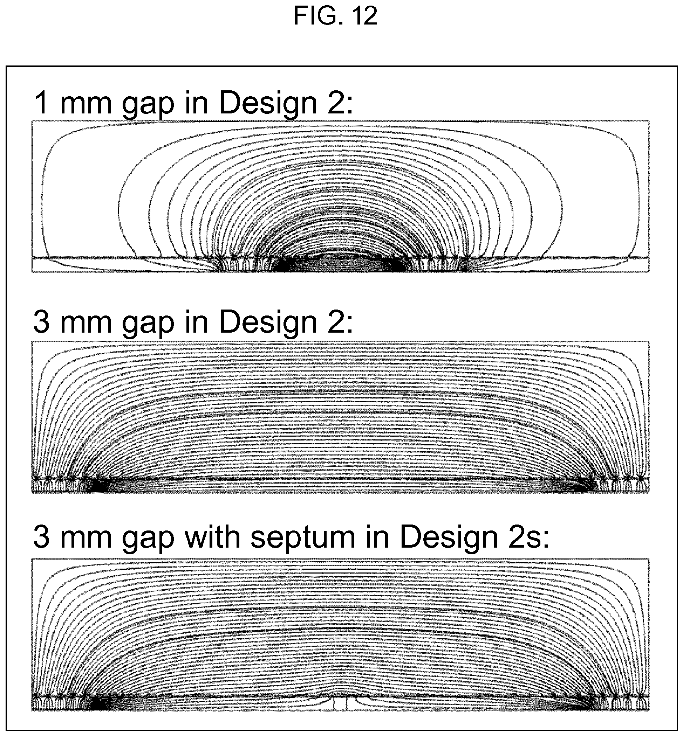

Figure 12 shows results of finite element simulations of electrode geometries with Design 2 with a 1 mm gap (top), Design 2 with a 3 mm gap (middle) and Design 2s with a 3 mm gap and a septum of width 0.1 mm (bottom). The current density distribution is shown as colour-map and field lines.

The top simulation of Design 2 with a 1 mm gap provides a basolateral resistance which is low and a large percentage of the current flows under the support membrane.

The middle simulation of Design 2 with a 3 mm gap provides a basolateral resistance which is increased. The total current flowing through the system is decreased and the percentage of the current flowing through the cells is increased.

The bottom simulation of Design 2s with a 0.1 mm wide insulating septum (i.e. dividing wall) between the primary electrodes is a design in which current is inhibited or prevented from flowing from electrode-to-electrode through the medium in the channel and so current is forced to flow through the culture. The basolateral resistance is the highest and the sensitivity is the highest. The cell-plane current density standard deviation is decreased to 3.41 A/m2 (27% of the mean). The current uniformity across the culture is also improved by provision of the septum.

## AIR-LIQUID INTERFACE EPITHELIAL CELL CULTURES

The advantage of a planar electrode geometry is the ability to measure the electrical properties of cells from one side only (basolateral compartment). The epithelial culture needs only a very small volume of electrolyte above the cells in the apical compartment. The sensitivity was examined as a function of the height of the electrolyte volume in the apical compartment. EIS measurements were performed on a confluent cell culture (Rc=1800 $\Omega$) and different volumes of medium were added to the apical compartment (0, 10, 20, 30, 40, 50 or 200 $\mu$l, leading to height of electrolyte in the apical compartment of 0, 0.3, 0.6, 1, 1.2,1.5 or 6 mm, respectively). To maximise the signal, the apical compartment resistance needs to be as low as possible. The electrical resistance Ra increases when culture medium is removed increasing the lower boundary of the measurement range. The sensitivity of the measurements decreases with increasing apical compartment resistance.

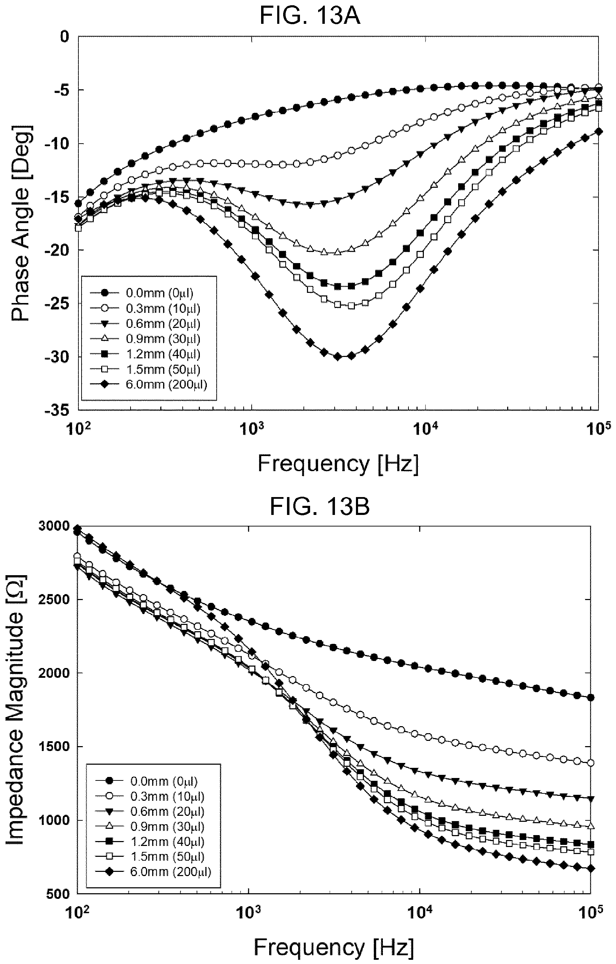

Figures 13A and 13B are graphs of electrical impedance amplitude/modulus and phase angle as a function of frequency for a confluent epithelial culture with different volumes of electrolyte in the apical compartment. At low frequencies, the impedance modulus is less affected by variation of the apical compartment resistance, Ra, due to the presence of the relatively high resistance, Rc, of the cell layer which is connected in series with Ra (see equivalence circuit) and dominates the measurement. The magnitude of the phase angle minimum at intermediate frequencies decreases progressively as less and less electrolyte is present. At high frequencies, the culture medium resistance, Rc, is short-circuited by the culture medium capacitance, Cc, and the impedance is affected by variations in the apical compartment resistance, Ra. In the phase angle plot of Figure 13B, the magnitude of the depth decreases when electrolyte is removed from the apical compartment.

Differences in the frequency-dependent response can be used to determine the sensitivity to electrolyte height variation in the apical compartment, defined as:

$$ S_{AV} = \frac{|Z_{100Hz}| - |Z_{100kHz}|}{|Z_{100kHz}|} $$

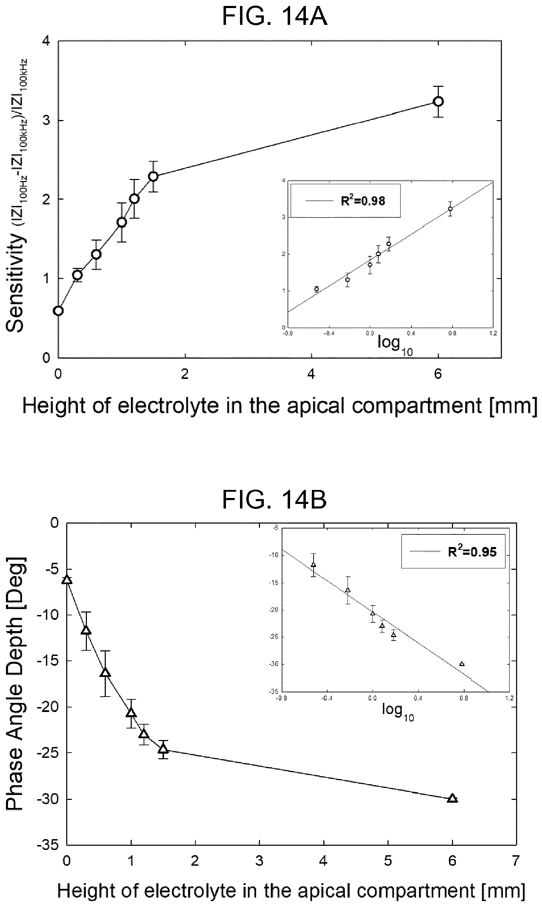

Figure 14A is a graph showing results of a measurement of sensitivity as given by impedance magnitude (i.e. modulus) as a function of height of the electrolyte volume in the apical compartment.

Figure 14B is a graph showing results of a measurement of sensitivity as given by impedance phase angle as a function of the height of the electrolyte volume in the apical compartment for the same measurements as shown in Figure 14A.

The sensitivity in impedance modulus (Figure 14A) and phase angle (Figure 14B) both depend on the amount of electrolyte in the apical compartment, since this changes the value of the apical compartment resistance, Ra.

Logarithmic regressions are shown in the insets to Figure 14A and Figure 14B. These both show a linear regression on a logarithmic scale. The best fit of the modulus regression has a value of $R^2$=0.986 and the phase regression of $R^2$=0.949. Error bars represent standard deviation (n=3). For the empty apical compartment the increase in low frequency impedance is due only to electrode polarization effects. For a volume of 200 $\mu$l the sensitivity reaches a plateau with a 3.38$\pm$0.05 fold increase between high and low frequency impedance modulus. From this data, the minimum volume of medium in the apical compartment necessary to measure a signal is approximately 10 $\mu$l.

## EPITHELIAL CULTURE GROWTH

Cell barrier resistance values were obtained by fitting impedance spectra (measured every 24 hours) of a growing culture to the equivalent electric circuit. Impedance spectra were measured over five days with 10, 20, 50 or 200 $\mu$l of medium in the apical compartment for each measurement. The extrapolated values of the equivalent resistance $R_c$ were compared with TER values.

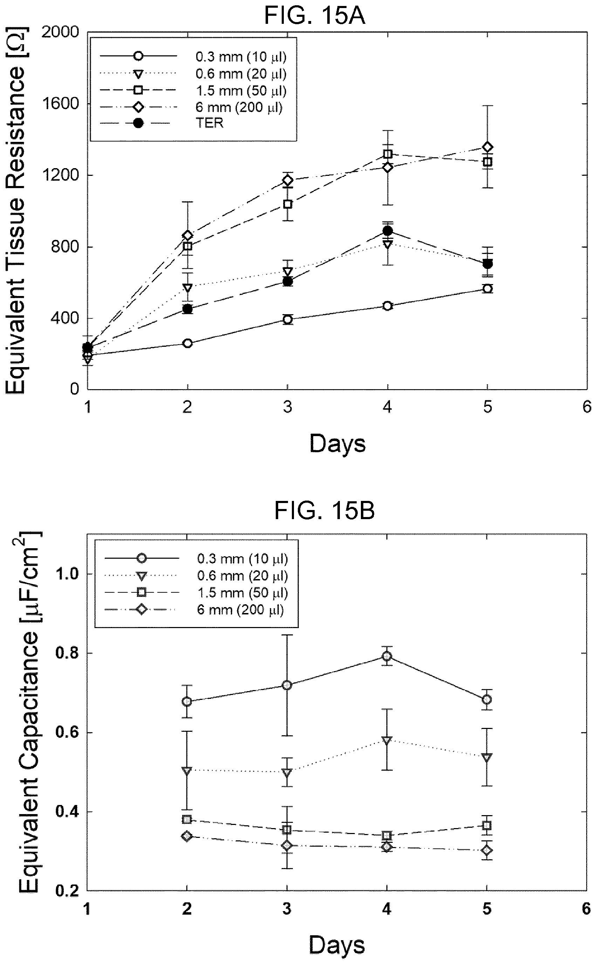

Figure 15A is a graph of resistance versus time showing the results of monitoring growth of epithelial cultures with different volumes of electrolyte in the apical compartment. Results are mean $\pm$ standard deviation (n=4) (n=4).

All experimental conditions show an increase in equivalent resistance over time which is comparable with the increase seen with the standard TER technique. With the planar electrodes it is possible to monitor the establishment of an epithelial barrier with only 0.3 mm (10 $\mu$l) of medium in the apical compartment. A height of 0.3 mm is compatible with the physiological amount of mucus produced by differentiated primary bronchial epithelial cells. The volume of medium needed is further lowered by inserting an insulating septum between the electrodes as discussed above in relation to Design 2s.

Figure 15B is a graph of capacitance versus time for the same results as Figure 15A obtained from electrical impedance spectroscopy. Results are mean $\pm$ standard deviation (n=4) (n=4). The equivalent capacitance becomes stable after the cells become confluent, i.e. have grown to occupy the entire area of the support membrane. The capacitance values are in the 50-110 nF range.

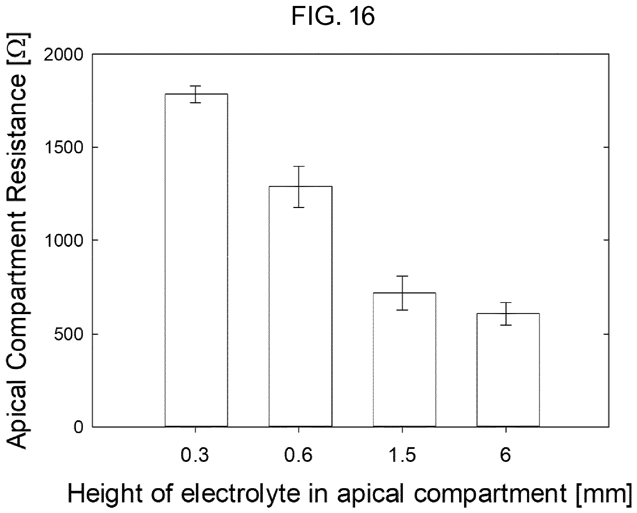

Figure 16 is a bar chart of fitted apical compartment resistance values, Ra, for different heights of medium in the apical compartment. The standard error across replicates was smaller than 15% (n=4). The fitted values for the apical compartment resistance have a logarithmic dependence on volume of electrolyte in the apical compartment. The apical volume resistance values ranged from 1780 $\Omega$ to 600 $\Omega$ with good consistency across the replicates (SD<15%).

## BARRIER DISRUPTION AT THE AIR-LIQUID INTERFACE

The capability of the device to monitor the epithelial barrier function at the air-liquid interface is demonstrated by measuring the change in the resistance of the epithelial barrier induced by stimulation of a differentiated culture of primary bronchial human cells with EGTA (a calcium chelator known to disrupt the barrier, through tight junction disorganisation). Impedance spectra were measured before and after EGTA stimulation either submerged or at the air-liquid interface. The extrapolated values of the equivalent resistance $R_c$ were compared with TER values.

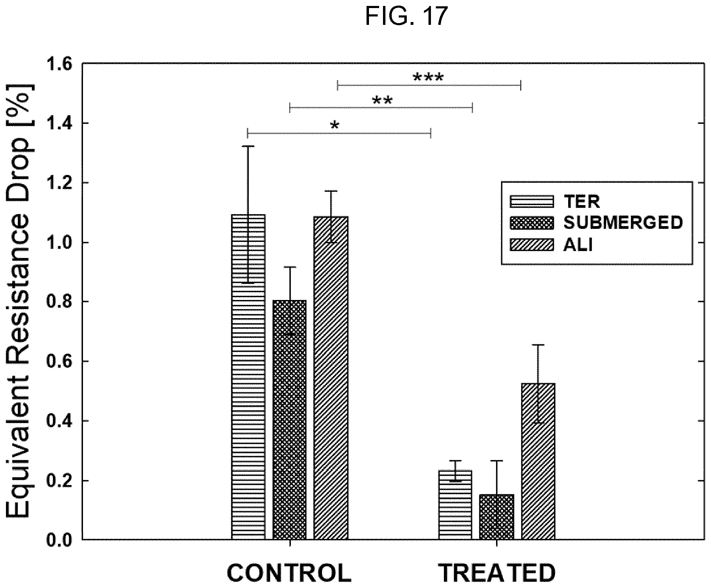

Figure 17 is a bar chart of fitted resistance values, Rc, showing the disruption induced by the EGTA stimulation. Compared to the control, the stimulated cultures shown a statistically significant (paired t-test) drop both for the submerged and the air-liquid interface measurement.

## MATERIALS AND METHODS

### FABRICATION

The planar electrodes were made of platinum electrodes and manufactured on a glass wafer (i.e. the substrate) in a clean room environment using standard photolithography techniques. Two layers of negative dry film resist (TMMF 55 $\mu$m thick from TOK (Tokyo Ohka Kogyo Co., Ltd.), Japan) were laminated and patterned using standard photolithography to create supporting spacers defining the reservoir around the electrodes area. Electrochemical deposition of platinum black (Pt-black) was performed on the electrodes using the procedure described elsewhere.

### FINITE ELEMENT ANALYSIS

Finite element simulations were performed using the electric currents module of COMSOL Multiphysics® (Comsol Inc., Sweden) 4.3b. The basolateral compartment was modelled as water with conductivity of 1.47 S/m. Electric insulation boundary conditions were applied to all the boundaries except the two electrodes which were set at ground and 0.05 V respectively. An extremely fine mesh was used and the steady state was analysed.

### CELL CULTURE

A human bronchial epithelial cell line, 16HBE 14o- was used and maintained in minimal essential medium (MEM) with Glutamax with addition of 10% foetal bovine serum (FBS), penicillin and streptomycin (all from Invitrogen, Paisley, UK). Cells were seeded with a density of $4.5 \times 10^5$ cells/cm2 into a well insert support with a diameter of 6.5 mm and pore size of 0.4 $\mu$m (Transwell®, Corning Life Sciences, Fisher Scientific UK). Volumes of 200 and 500 $\mu$l of medium were added in the apical and basolateral compartments respectively, and media were changed during the electrical measurement.

### MEASUREMENTS

Electrical impedance spectroscopy (EIS) measurements were performed using the two electrodes configuration of an Alpha-A impedance analyser (Novocontrol Technologies, Germany). A frequency sweep was performed between 100 Hz and 100 kHz, with an applied voltage of 0.05 V(rms). Prior to the EIS measurement, the medium in the apical compartment was replaced with different volumes (200 $\mu$l, 50 $\mu$l, 20 $\mu$l, 10 $\mu$l) of fresh medium at roomtemperature. Each Transwell® support was placed on top of the planar electrodes only for the duration of the measurement ($\approx$30s). Trans-epithelial electrical resistance (TER) measurements were performed using chopstick electrodes connected to an EVOM2 Epithelial Volt-ohm-meter (World Precision Instruments).

### COMPLEX ELECTRICAL IMPEDANCE FITTING

A custom script was developed in Matlab (@2015 The MathWorks) to fit the complex electrical impedance data with the equivalent electric circuit model. The objective function to be minimized during the fitting was defined as:

$$\varphi = \sum_i \sqrt{\left(\frac{re\left(Z^{exp}(f_i)\right)-re\left(Z^{teo}(f_i,p)\right)}{\left|\left(Z^{exp}(f_i)\right)\right|}\right)^2 + \left(\frac{im\left(Z^{exp}(f_i)\right)-im\left(Z^{teo}(f_i,p)\right)}{\left|\left(Z^{exp}(f_i)\right)\right|}\right)^2}$$

The absolute value of the impedance was used as the normalizing factor. The impedance spectrum at the time of cell seeding was used to obtain the parameters relative to the electrode polarisation and to the basolateral resistance; these parameters were fixed for the subsequent fittings. Impedance spectra relative to successive time points were fitted, and values for the parameters relative to the cell culture and to the apical compartment resistance were obtained.

**Claims**

1.  A microfluidic device comprising:

    a device body;
    a porous support extending across an internal volume in the device body to define a partition between an upper, apical compartment that is bounded on its lower side by the upper surface of the porous support and a lower, basolateral compartment that is bounded on its upper side by a lower surface of the porous support and on its lower side by an internal surface of the device body;
    an inlet and an outlet arranged in the device body that provide a path for fluid flow of a liquid medium through a flow channel that includes the basolateral compartment;
    a first electrode and a second electrode arranged on the internal surface of the device body on the lower side of the flow channel, the first and second electrodes being electrically connected to respective internal ends of respective electrically conductive paths, the electrically conductive paths having respective external ends that provide respective external contacts via which a bias voltage is applicable between the first and second electrodes to create an electric field to induce an electrical current to flow between the first and second electrodes at least in part via the cell layer when a liquid medium is present in the flow channel and when a cell layer has been grown on the porous support; and
    a septum arranged in the flow channel between the first electrode and the second electrode, the septum extending at least part way between the internal surface of the device body and the porous support, the septum being made of a material that acts as an electrical barrier to the electric field, so that the septum is configured to inhibit electrical current flowing directly from the first electrode to the second electrode through the liquid medium and to promote electrical current flowing from the first electrode to the second electrode via the cell layer.

2.  The device of claim 1, wherein the flow channel is shaped and arranged to provide a predominant flow direction for the liquid medium passing through it, and the septum is arranged substantially aligned with the predominant flow direction

3.  The device of claim 1 or 2, wherein the septum extends from the internal surface of the device body to the lower surface of the porous support.

4.  A microfluidic device comprising:

    a device body;
    a porous support extending across an internal volume in the device body to define a partition between an upper, apical compartment that is bounded on its lower side by the upper surface of the porous support and a lower, basolateral compartment that is bounded on its upper side by a lower surface of the porous support and on its lower side by an internal surface of the device body;
    an inlet and an outlet arranged in the device body that provide a path for fluid flow of a liquid medium through a flow channel that includes the basolateral compartment;
    a first electrode and a second electrode arranged on the internal surface of the device body on the lower side of the flow channel, the first and second electrodes being electrically connected to respective internal ends of respective electrically conductive paths, the electrically conductive paths having respective external ends that provide respective external contacts via which a bias voltage is applicable between the first and second electrodes to create an electric field to induce an electrical current to flow between the first and second electrodes at least in part via the cell layer when a liquid medium is present in the flow channel and when a cell layer has been grown on the porous support, wherein the fluid flow in the basolateral compartment has a flow direction, and the first and second electrodes have a minimum separation inside the basolateral compartment of 26% of a maximum width of the basolateral compartment.

5. The device of claim 4, wherein the combined area of the first and second electrodes inside the basolateral compartment is at least 20% of the area of the basolateral compartment.

6. The device of claim 5, wherein the combined area of the first and second electrodes inside the basolateral compartment is less than 30% of the area of the basolateral compartment.

7. The device of claim 4 or 5, wherein the basolateral compartment has a truncated cylindrical shape.

8. The device of any of claims 4 to 7, wherein the first and second electrodes have an approximately equal separation across the basolateral compartment.

9. The device of any preceding claim, further comprising:
a third electrode and a fourth electrode arranged in the flow channel, the third and fourth electrodes being electrically connected to respective internal ends of respective electrically conductive paths, the electrically conductive paths having respective external ends that provide respective external contacts via which a bias voltage is applicable between the third and fourth electrodes to create an electric field to induce an electrical current to flow between the third and fourth electrodes when a liquid medium is present in the flow channel, the third and fourth electrodes being so arranged in the flow channel that current passes between them via the liquid medium and without passing via a cell layer grown on the porous support.

10. The device of claim 9, wherein the third and fourth electrodes are arranged in the flow channel outside the basolateral compartment.

11. A system comprising the microfluidic device of any of claims 1 to 10 in combination with a holder for the microfluidic device to facilitate liquid-tight connections for injection and removal of liquids into and out of the microfluidic device and to establish electrical connections to the microfluidic device's electrodes.

12. A method of using the microfluidic device of any of claims 1 to 10:

to supply a liquid medium through the flow channel to the basolateral compartment;
to grow a cell layer on the porous support; and
to use the electrodes to take measurements of the cell layer at different stages of its growth.

**Patentansprüche**

1. Mikrofluidische Vorrichtung, umfassend:

einen Vorrichtungskörper;
einen porösen Träger, der sich über ein internes Volumen in dem Vorrichtungskörper erstreckt, um eine Partition zwischen einem oberen, apikalen Kompartiment, das auf seiner unteren Seite von der oberen Oberfläche des porösen Trägers begrenzt ist, und einem unteren, basolateralen Kompartiment, das auf seiner oberen Seite von einer unteren Oberfläche des porösen Trägers und auf seiner unteren Seite von einer internen Oberfläche des Vorrichtungskörpers begrenzt ist, zu definieren;
einen Einlass und einen Auslass, die in dem Vorrichtungskörper angeordnet sind, die einen Weg für Fluidfluss eines flüssigen Mediums durch einen Flusskanal, der das basolaterale Kompartiment beinhaltet, bereitstellen;
eine erste Elektrode und eine zweite Elektrode, die auf der internen Oberfläche des Vorrichtungskörpers auf der unteren Seite des Flusskanals angeordnet sind, wobei die erste und zweite Elektrode elektrisch mit jeweiligen internen Enden jeweiliger elektrisch leitender Wege verbunden sind, wobei die elektrisch leitenden Wege jeweilige externe Enden aufweisen, die jeweilige externe Kontakte bereitstellen, über die eine Vorspannung zwischen der ersten und zweiten Elektrode anlegbar ist, um ein elektrisches Feld zu erzeugen, um das Fließen eines elektrischen Stroms zwischen der ersten und zweiten Elektrode mindestens zum Teil über die Zellschicht zu induzieren, wenn ein flüssiges Medium in dem Flusskanal vorliegt und wenn eine Zellschicht auf dem porösen Träger gezüchtet wurde; und

ein Septum, das in dem Flusskanal zwischen der ersten Elektrode und der zweiten Elektrode angeordnet ist, wobei sich das Septum mindestens einen Teil des Wegs zwischen der internen Oberfläche des Vorrichtungskörpers und dem porösen Träger erstreckt, wobei das Septum aus einem Material besteht, das als elektrische Barriere gegen das elektrische Feld wirkt, so dass das Septum konfiguriert ist zur Hemmung des Fließens von elektrischem Strom direkt von der ersten Elektrode zu der zweiten Elektrode durch das flüssige Medium und zur Förderung des Fließens von elektrischem Strom von der ersten Elektrode zu der zweiten Elektrode über die Zellschicht.

2. Vorrichtung nach Anspruch 1, wobei der Flusskanal geformt und angeordnet ist, um eine vorherrschende Flussrichtung für das durch ihn verlaufende flüssige Medium bereitzustellen, und das Septum im Wesentlichen mit der vorherrschenden Flussrichtung ausgerichtet angeordnet ist.

3. Vorrichtung nach Anspruch 1 oder 2, wobei sich das Septum von der internen Oberfläche des Vorrichtungskörpers zu der unteren Oberfläche des porösen Trägers erstreckt.

4. Mikrofluidische Vorrichtung, umfassend:

einen Vorrichtungskörper;
einen porösen Träger, der sich über ein internes Volumen in dem Vorrichtungskörper erstreckt, um eine Partition zwischen einem oberen, apikalen Kompartiment, das auf seiner unteren Seite von der oberen Oberfläche des porösen Trägers begrenzt ist, und einem unteren, basolateralen Kompartiment, das auf seiner oberen Seite von einer unteren Oberfläche des porösen Trägers und auf seiner unteren Seite von einer internen Oberfläche des Vorrichtungskörpers begrenzt ist, zu definieren;
einen Einlass und einen Auslass, die in dem Vorrichtungskörper angeordnet sind, die einen Weg für Fluidfluss eines flüssigen Mediums durch einen Flusskanal, der das basolaterale Kompartiment beinhaltet, bereitstellen;
eine erste Elektrode und eine zweite Elektrode, die auf der internen Oberfläche des Vorrichtungskörpers auf der unteren Seite des Flusskanals angeordnet sind, wobei die erste und zweite Elektrode elektrisch mit jeweiligen internen Enden jeweiliger elektrisch leitender Wege verbunden sind, wobei die elektrisch leitenden Wege jeweilige externe Enden aufweisen, die jeweilige externe Kontakte bereitstellen, über die eine Vorspannung zwischen der ersten und zweiten Elektrode anlegbar ist, um ein elektrisches Feld zu erzeugen, um das Fließen eines elektrischen Stroms zwischen der ersten und zweiten Elektrode mindestens zum Teil über die Zellschicht zu induzieren, wenn ein flüssiges Medium in dem Flusskanal vorliegt und wenn eine Zellschicht auf dem porösen Träger gezüchtet wurde, wobei der Fluidfluss in dem basolateralen Kompartiment eine Flussrichtung aufweist und die erste und zweite Elektrode eine minimale Separation innen in dem basolateralen Kompartiment von 26 % einer maximalen Breite des basolateralen Kompartiments aufweisen.

5. Vorrichtung nach Anspruch 4, wobei die kombinierte Fläche der ersten und zweiten Elektrode innen in dem basolateralen Kompartiment mindestens 20 % der Fläche des basolateralen Kompartiments beträgt.

6. Vorrichtung nach Anspruch 5, wobei die kombinierte Fläche der ersten und zweiten Elektrode innen in dem basolateralen Kompartiment weniger als 30 % der Fläche des basolateralen Kompartiments beträgt.

7. Vorrichtung nach Anspruch 4 oder 5, wobei das basolaterale Kompartiment eine Zylinderstumpfform aufweist.

8. Vorrichtung nach einem der Ansprüche 4 bis 7, wobei die erste und zweite Elektrode eine ungefähr gleiche Separation über das basolaterale Kompartiment aufweisen.

9. Vorrichtung nach einem vorhergehenden Anspruch, ferner umfassend:
eine dritte Elektrode und eine vierte Elektrode, die in dem Flusskanal angeordnet sind, wobei die dritte und vierte Elektrode elektrisch mit jeweiligen internen Enden jeweiliger elektrisch leitender Wege verbunden sind, wobei die elektrisch leitenden Wege jeweilige externe Enden aufweisen, die jeweilige externe Kontakte bereitstellen, über die eine Vorspannung zwischen der dritten und vierten Elektrode anlegbar ist, um ein elektrisches Feld zu erzeugen, um das Fließen eines elektrischen Stroms zwischen der dritten und vierten Elektrode zu induzieren, wenn ein flüssiges Medium in dem Flusskanal vorliegt, wobei die dritte und vierte Elektrode so in dem Flusskanal angeordnet sind, dass Strom zwischen ihnen über das flüssige Medium, und ohne über eine auf dem porösen Träger gezüchtete Zellschicht zu verlaufen, verläuft.

10. Vorrichtung nach Anspruch 9, wobei die dritte und vierte Elektrode in dem Flusskanal außerhalb des basolateralen Kompartiments angeordnet sind.

11. System, umfassend die mikrofluidische Vorrichtung nach einem der Ansprüche 1 bis 10 in Kombination mit einem Halter für die mikrofluidische Vorrichtung, um flüssigkeitsdichte Verbindungen zur Injektion und Entnahme von Flüssigkeiten in die und aus der mikrofluidische(n) Vorrichtung zu ermöglichen und elektrische Verbindungen zu den Elektroden der mikrofluidischen Vorrichtung herzustellen.

12. Verfahren zur Verwendung der mikrofluidischen Vorrichtung nach einem der Ansprüche 1 bis 10:

zum Zuführen eines flüssigen Mediums durch den Flusskanal zu dem basolateralen Kompartiment;
zum Züchten einer Zellschicht auf dem porösen Träger; und
zum Benutzen der Elektroden zum Vornehmen von Messungen der Zellschicht in verschiedenen Phasen ihres Wachstums.

**Revendications**

1. Dispositif microfluidique comprenant :

un corps de dispositif ;
un support poreux s'étendant en travers d'un volume interne dans le corps de dispositif pour définir une cloison entre un compartiment apical supérieur qui est délimité sur son côté inférieur par la surface supérieure du support poreux et un compartiment basolatéral inférieur qui est délimité sur son côté supérieur par une surface inférieure du support poreux et sur son côté inférieur par une surface interne du corps de dispositif ;
une entrée et une sortie disposées dans le corps de dispositif qui fournissent un chemin pour l'écoulement fluidique d'un milieu liquide à travers un canal d'écoulement qui comporte le compartiment basolatéral ;
une première électrode et une deuxième électrode disposées sur la surface interne du corps de dispositif sur le côté inférieur du canal d'écoulement, les première et deuxième électrodes étant connectées électriquement à des extrémités internes respectives de chemins électriquement conducteurs respectifs, les chemins électriquement conducteurs présentant des extrémités externes respectives qui fournissent des contacts externes respectifs par l'intermédiaire desquels une tension de polarisation peut être appliquée entre les première et deuxième électrodes pour créer un champ électrique afin d'induire le passage d'un courant électrique entre les première et deuxième électrodes au moins en partie par l'intermédiaire de la couche cellulaire quand un milieu liquide est présent dans le canal d'écoulement et quand une couche cellulaire a été développée sur le support poreux ; et
un septum disposé dans le canal d'écoulement entre la première électrode et la deuxième électrode, le septum s'étendant au moins à mi-chemin entre la surface interne du corps de dispositif et le support poreux, le septum étant réalisé en un matériau qui fait office de barrière électrique au champ électrique, de telle sorte que le septum soit configuré pour inhiber le passage d'un courant électrique directement de la première électrode à la deuxième électrode à travers le milieu liquide et favoriser le passage d'un courant électrique de la première électrode à la deuxième électrode par l'intermédiaire de la couche cellulaire.

2. Dispositif selon la revendication 1, dans lequel le canal d'écoulement est conformé et disposé de manière à fournir une direction d'écoulement prédominante pour le milieu liquide qui le traverse, et le septum est disposé sensiblement en alignement avec la direction d'écoulement prédominante.

3. Dispositif selon la revendication 1 ou 2, dans lequel le septum s'étend depuis la surface interne du corps de dispositif jusqu'à la surface inférieure du support poreux.

4. Dispositif microfluidique comprenant :

un corps de dispositif ;
un support poreux s'étendant en travers d'un volume interne dans le corps de dispositif pour définir une cloison entre un compartiment apical supérieur qui est délimité sur son côté inférieur par la surface supérieure du support poreux et un compartiment basolatéral inférieur qui est délimité sur son côté supérieur par une surface inférieure du support poreux et sur son côté inférieur par une surface interne du corps de dispositif ;
une entrée et une sortie disposées dans le corps de dispositif qui fournissent un chemin pour l'écoulement fluidique d'un milieu liquide à travers un canal d'écoulement qui comporte le compartiment basolatéral ;

une première électrode et une deuxième électrode disposées sur la surface interne du corps de dispositif sur le côté inférieur du canal d'écoulement, les première et deuxième électrodes étant connectées électriquement à des extrémités internes respectives de chemins électriquement conducteurs respectifs, les chemins électriquement conducteurs présentant des extrémités externes respectives qui fournissent des contacts externes respectifs par l'intermédiaire desquels une tension de polarisation peut être appliquée entre les première et deuxième électrodes pour créer un champ électrique afin d'induire le passage d'un courant électrique entre les première et deuxième électrodes au moins en partie par l'intermédiaire de la couche cellulaire quand un milieu liquide est présent dans le canal d'écoulement et quand une couche cellulaire a été développée sur le support poreux, dans lequel l'écoulement fluidique dans le compartiment basolatéral a une direction d'écoulement, et les première et deuxième électrodes ont une séparation minimale à l'intérieur du compartiment basolatéral de 26 % d'une largeur maximale du compartiment basolatéral.

5. Dispositif selon la revendication 4, dans lequel l'aire combinée des première et deuxième électrodes à l'intérieur du compartiment basolatéral est au moins 20 % de l'aire du compartiment basolatéral.

6. Dispositif selon la revendication 5, dans lequel l'aire combinée des première et deuxième électrodes à l'intérieur du compartiment basolatéral est inférieure à 30 % de l'aire du compartiment basolatéral.

7. Dispositif selon la revendication 4 ou 5, dans lequel le compartiment basolatéral a une forme cylindrique tronquée.

8. Dispositif selon l'une quelconque des revendications 4 à 7, dans lequel les première et deuxième électrodes présentent une séparation approximativement égale en travers du compartiment basolatéral.

9. Dispositif selon n'importe quelle revendication précédente, comprenant en outre :
une troisième électrode et une quatrième électrode disposées dans le canal d'écoulement, les troisième et quatrième électrodes étant connectées électriquement à des extrémités internes respectives de chemins électriquement conducteurs respectifs, les chemins électriquement conducteurs présentant des extrémités externes respectives qui fournissent des contacts externes respectifs par l'intermédiaire desquels une tension de polarisation peut être appliquée entre les troisième et quatrième électrodes pour créer un champ électrique afin d'induire le passage d'un courant électrique entre les troisième et quatrième électrodes quand un milieu liquide est présent dans le canal d'écoulement, les troisième et quatrième électrodes étant disposées dans le canal d'écoulement de telle sorte que le courant passe entre elles par l'intermédiaire du milieu liquide sans passer par l'intermédiaire d'une couche cellulaire développée sur le support poreux.

10. Dispositif selon la revendication 9, dans lequel les troisième et quatrième électrodes sont disposées dans le canal d'écoulement à l'extérieur du compartiment basolatéral.

11. Système comprenant le dispositif microfluidique selon l'une quelconque des revendications 1 à 10 en combinaison avec un support pour le dispositif microfluidique afin de faciliter des connexions étanches aux liquides pour l'injection et l'élimination de liquides dans et hors du dispositif microfluidique et d'établir des connexions électriques avec les électrodes du dispositif microfluidique.

12. Procédé d'utilisation du dispositif microfluidique selon l'une quelconque des revendications 1 à 10 :

pour fournir un milieu liquide à travers le canal d'écoulement jusqu'au compartiment basolatéral ;
pour développer une couche cellulaire sur le support poreux ; et
pour utiliser les électrodes afin de réaliser des mesures de la couche cellulaire à différents stades de son développement.

# FIG. 1A
## (prior art)

TRANSWELL®

ELECTRODES

WELL PLATE

APICAL
COMPARTMENT

EPITHELIAL
CELLS

BASOLATERAL
COMPARTMENT

NANOPOROUS
SUPPORT

# FIG. 1B
## (Hediger et al 2000)

upper electrodes

porous
membrane

upper wafer

lower wafer

channel

substrate

lower electrodes

# FIG. 1C
## (Sun et al 2010)

# FIG. 1D
## (Odijk et al 2015)

## FIG. 1E
### (Henry et al 2017)

# FIG. 2A

# FIG. 2B

# FIG. 3A

# FIG. 3B

FIG. 3C

FIG. 3D

## FIG. 3E

ELECTRIC CONNECTORS

FLUIDIC SEAL (O-RING)

COOLING DEVICE

SYRINGE PUMP

VALVE

WASTE COLLECTOR

CHIP

HOLDER

SAMPLE COLLECTOR

INCUBATOR

## FIG. 4

APICAL COMPARTMENT

CELL MEMBRANE

SUPPORT MEMBRANE

BASOLATERAL COMPARTMENT (FLOW CHANNEL)

PRIMARY ELECTRODE

$R_a$

$R_c$

$C_c$

$R_s$

$R_b$

CPE

CELL NUCLEUS

CYTOSOL

TIGHT JUNCTION

PORE

# FIG. 5A

# FIG. 5B

## FIG. 6

## FIG. 7

# FIG. 8

Design 0:
Sun *et al.* (2010)

Design 1

Design 2

# FIG. 9

FIG. 10

# FIG. 11

**KEY**: white arrows indicate preferred liquid medium flow direction

## FIG. 12

1 mm gap in Design 2:

3 mm gap in Design 2:

3 mm gap with septum in Design 2s:

## FIG. 13A

## FIG. 13B

## FIG. 14A

Sensitivity $(|Z|_{100Hz}-|Z|_{100kHz})/|Z|_{100kHz}$ vs Height of electrolyte in the apical compartment [mm]

## FIG. 14B

Phase Angle Depth [Deg] vs Height of electrolyte in the apical compartment [mm]

## FIG. 15A

## FIG. 15B

FIG. 16

FIG. 17

EP 3 746 784 B1

## REFERENCES CITED IN THE DESCRIPTION

### Patent documents cited in the description

- WO 2017096297 A1

### Non-patent literature cited in the description

- **ODIJK, VAN DER MEER, LEVNER, KIM, VAN DER HELM, SEGERINK, FRIMAT, HAMILTON, INGBER,VAN DEN BERG.** Measuring direct current trans-epithelial electrical resistance in organ-on-a-chip. *Lab on a Chip,* 07 February 2015, vol. 15 (3), 745-52
- **HEDIGER, FONTANNAZ, SAYAH, HUNZIKER AND GIJS.** Biosystem for the culture and characterisation of epithelial cell tissues. *Sensors and Actuators B: Chemical,* 20 April 2000, vol. 63, 63-73
- **SUN, SWINDLE, COLLINS, HOLLOWAY, DAVIES AND MORGAN.** On-chip epithelial barrier function assays using electrical impedance spectroscopy. *Lab on a Chip,* 08 April 2010, vol. 10 (12), 1611-1617
- **OLIVIER Y. F. HENRY, REMI VILLENAVE, MICHAEL J. CRONCE, WILLIAM D. LEINEWEBER.** Organs-on-chips with integrated electrodes for trans-epithelial electrical resistance. *Lab on a Chip,* 26 May 2017, vol. 17, 2264-71